# EUROPEAN PATENT SPECIFICATION

(11) **EP 3 306 693 B1**
(45) Date of publication and mention of the grant of the patent: **08.04.2020**
(21) Application number: 16803039.3
(22) Date of filing: 17.05.2016
(51) Int. Cl.: C09K 11/02, H01L 51/50, C07D 209/86, C07D 487/04, C09K 11/06

(54) **ORGANIC ELECTROLUMINESCENT ELEMENT**
ORGANISCHES ELEKTROLUMINESZENTES ELEMENT
ÉLÉMENT ÉLECTROLUMINESCENT (EL) ORGANIQUE

(30) Priority: 29.05.2015 JP 2015110312
(43) Date of publication of application: 11.04.2018
(73) Proprietor: NIPPON STEEL Chemical & Material Co., Ltd., Tokyo 103-0027 (JP)
(72) Inventor: TADA Masashi, Kitakyushu-shi Fukuoka 804-8503 (JP); KAI Takahiro, Kitakyushu-shi Fukuoka 804-8503 (JP); UEDA Tokiko, Kitakyushu-shi Fukuoka 804-8503 (JP); OGAWA Junya, Kitakyushu-shi Fukuoka 804-8503 (JP); NOGUCHI Katsuhide, Kitakyushu-shi Fukuoka 804-8503 (JP); HOTTA Masanori, Kitakyushu-shi Fukuoka 804-8503 (JP)
(74) Representative: Vossius & Partner Patentanwälte Rechtsanwälte mbB
(86) International application number: PCT/JP2016/064658
(87) International publication number: WO 2016/194604

(56) References cited:
- EP-A1- 2 866 273
- WO-A1-2013/062075
- WO-A1-2013/062075
- JP-A- 2015 097 201
- KR-A- 20150 006 722
- US-A1- 2015 001 488
- US-A1- 2015 001 488

## Description

### Technical Field

The present invention relates to an organic electroluminescent device (referred to as "organic EL device").

### Background Art

When a voltage is applied to an organic EL device, a hole is injected from an anode into a light-emitting layer, and an electron is injected from a cathode into the layer. Then, in the light-emitting layer, the hole and the electron thus injected recombine to produce an exciton. At this time, according to the statistical law of electron spins, singlet excitons and triplet excitons are produced at a ratio of 1:3. The internal quantum efficiency of a fluorescent emission-type organic EL device using light emission by a singlet exciton is said to be at most 25%. Meanwhile, it has been known that the internal quantum efficiency of a phosphorescent emission-type organic EL device using light emission by a triplet exciton can be improved to 100% when intersystem crossing from a singlet exciton is efficiently performed.

However, the lengthening of the lifetime of the phosphorescent emission-type organic EL device has been a technical problem.

Further, a high-efficiency organic EL device utilizing delayed fluorescence has been recently developed. In, for example, Patent Literature 1, there is a disclosure of an organic EL device utilizing a triplet-triplet fusion (TTF) mechanism serving as one of the delayed fluorescence mechanisms. The TTF mechanism utilizes a phenomenon in which a singlet exciton is produced by collision between two triplet excitons, and is considered to be capable of improving internal quantum efficiency to 40% in theory. However, a further improvement in efficiency has been required because the efficiency of the device is lower than that of a phosphorescent light-emitting organic EL device.

Meanwhile, in Patent Literature 2, there is a disclosure of an organic EL device utilizing a thermally activated delayed fluorescence (TADF) mechanism. The TADF mechanism utilizes a phenomenon in which inverse intersystem crossing from a triplet exciton to a singlet exciton occurs in a material having a small energy difference between a singlet level and a triplet level, and is considered to be capable of improving internal quantum efficiency to 100% in theory. However, a further improvement in lifetime characteristic has been required as in a phosphorescent light-emitting device.

### Citation List

### Patent Literature

[PTL 1] WO 2010/134350 A1
[PTL 2] WO 2011/070963 A1
[PTL 3] WO 2008/056746 A1
[PTL 4] JP 2003-133075 A
[PTL 5] WO 2013/062075 A1
[PTL 6] US 2014/0374728 A1
[PTL 7] US 2014/0197386 A1
[PTL 8] US 2015/0001488 A1
[PTL 9] WO 2011/136755 A1

In Patent Literature 3, there is a disclosure of the use of an indolocarbazole compound as a host material. In Patent Literature 4, there is a disclosure of the use of a biscarbazole compound as a host material.

In each of Patent Literatures 5 and 6, there is a disclosure of the use of a biscarbazole compound as a mixed host. In each of Patent Literatures 7 and 8, there is a disclosure of the use of an indolocarbazole compound and a biscarbazole compound as a mixed host.

In Patent Literature 9, there is a disclosure of the use of a host material obtained by preliminarily mixing a plurality of hosts including an indolocarbazole compound.

However, each of the literatures cannot be said to be sufficient, and hence a further improvement has been desired.

### Summary of Invention

In order to apply an organic EL device to a display device, such as a flat panel display, or a light source, the luminous efficiency of the device needs to be improved, and at the same time, stability at the time of its driving needs to be sufficiently secured. In view of the above-mentioned present circumstances, an object of the present invention is to provide a practically useful organic EL device having high efficiency and high driving stability while having a low driving voltage.

According to one embodiment of the present invention, there is provided an organic EL device, including one or more light-emitting layers between an anode and a cathode opposite to each other, inwhichat least one of the light-emitting layers contains a first host selected from compounds each represented by the following general formula (1), a second host selected from compounds each represented by the following general formula (2), and a light-emitting dopant material: 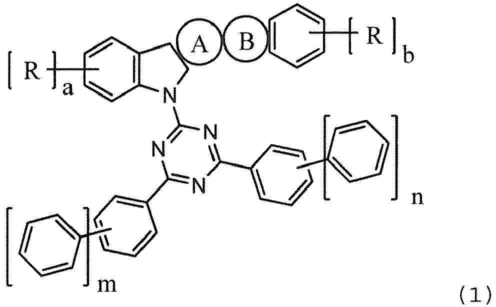 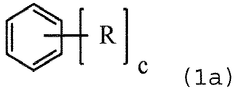 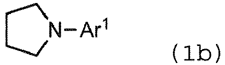 where:
a ring A includes an aromatic hydrocarbon ring represented by the formula (1a), a ring B includes a heterocycle represented by the formula (1b), and the ring A and the ring B are each fused with an adjacent ring at arbitrary positions;
Ar¹ represents a phenyl group, a biphenyl group, or a terphenyl group;
R's each independently represents an aliphatic hydrocarbon group having 1 to 10 carbon atoms, an aromatic hydrocarbon group having 6 to 10 carbon atoms, or an aromatic heterocyclic group having 3 to 12 carbon atoms;
a, b, and c each represent a substitution number, and each independently represent an integer of from 0 to 3; and
m and n each represent a substitution number, and each independently represent an integer of from 0 to 2, and m+n represents an integer of 1 or more; 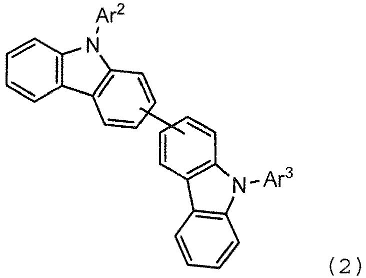
where Ar² and Ar³ each represent an aromatic hydrocarbon group having 6 to 14 carbon atoms, or a group obtained by linking two or three of the aromatic hydrocarbon groups, and at least one of Ar² or Ar³ represents a fused aromatic hydrocarbon group.

A preferred mode of the general formula (2) is the general formula (3).

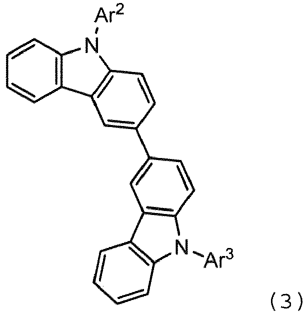

Ar² and Ar³ of the general formula (3) are identical in meaning to Ar² and Ar³ of the general formula (2). In addition, Ar² more preferably represents one of a naphthyl group and a phenanthryl group.

The first host and the second host are preferably used after having been preliminarily mixed before vapor deposition. In addition, it is preferred that a difference in 50% weight reduction temperature between the first host and the second host be 20°C or less, or the ratio of the first host be more than 20 wt% and less than 55 wt% with respect to the total of the first host and the second host.

The light-emitting dopant material may be a phosphorescent light-emitting dopant material, a fluorescent light-emitting dopant material, or a thermally activated delayed fluorescent light-emitting dopant material. An example of the phosphorescent light-emitting dopant material is an organometallic complex containing at least one metal selected from ruthenium, rhodium, palladium, silver, rhenium, osmium, iridium, platinum, and gold.

In addition, it is preferred that the organic EL device further include a hole-blocking layer adjacent to the light-emitting layers, the hole-blocking layer containing a compound represented by the general formula (1).

The organic EL device of the present invention can be an organic EL device having a low driving voltage, high luminous efficiency, and a long lifetime because the device contains a plurality of specific host materials in a light-emitting layer thereof.

### Brief Description of Drawings

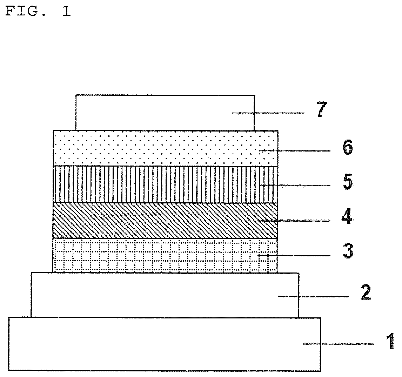

FIG. 1 is a schematic sectional view for illustrating an example of an organic EL device.

### Description of Embodiments

An organic EL device of the present invention includes one or more light-emitting layers between an anode and a cathode opposite to each other, and at least one of the light-emitting layers contains a first host, a second host, and a light-emitting dopant material. The first host is a compound represented by the general formula (1), and the second host is a compound represented by the general formula (2). The organic EL device includes an organic layer formed of a plurality of layers between the anode and the cathode opposite to each other. At least one of the plurality of layers is a light-emitting layer, and a plurality of light-emitting layers maybe present. Inaddition, the light-emitting layers are desirably formed of deposited layers produced by vacuum deposition.

The general formula (1) is described.

A ring A is an aromatic hydrocarbon ring represented by the formula (1a), a ring B is a heterocycle represented by the formula (1b), and the ring A and the ring B are each fused with an adjacent ring at arbitrary positions.

Ar¹ represents a phenyl group, a biphenyl group, or a terphenyl group, preferably a phenyl group or a biphenyl group, more preferably a phenyl group. Here, the biphenyl group is a group represented by -Ph-Ph, and the terphenyl group is a group represented by -Ph-Ph-Ph or -Ph(-Ph)-Ph. Ph represents a phenyl group or a phenylene group.

R's each independently represents an aliphatic hydrocarbon group having 1 to 10 carbon atoms, an aromatic hydrocarbon group having 6 to 10 carbon atoms, or an aromatic heterocyclic group having 3 to 12 carbon atoms, preferably an aliphatic hydrocarbon group having 1 to 8 carbon atoms, a phenyl group, or an aromatic heterocyclic group having 3 to 9 carbon atoms, more preferably an aliphatic hydrocarbon group having 1 to 6 carbon atoms, a phenyl group, or an aromatic heterocyclic group having 3 to 6 carbon atoms.

Specific examples of the aliphatic hydrocarbon group having 1 to 10 carbon atoms include methyl, ethyl, propyl, butyl, pentyl, hexyl, heptyl, octyl, nonyl, and decyl.

Specific examples of the aromatic hydrocarbon group having 6 to 10 carbon atoms or the aromatic heterocyclic group having 3 to 12 carbon atoms include aromatic groups each produced by removing one H atom from benzene, naphthalene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, benzothiadiazole, dibenzofuran, dibenzothiophene, dibenzoselenophene, or carbazole. Preferred example thereof include aromatic groups each produced from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, or benzothiadiazole. More preferred examples thereof include aromatic groups each produced from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, or oxadiazole.

a, b, and c each represent a substitution number, and each independently represent an integer of from 0 to 3, preferably an integer of 0 or 1. m and n each represent a substitution number, and each independently represent an integer of from 0 to 2, preferably an integer of 0 or 1. m+n represents an integer of 1 or more, preferably an integer of 1, 2, or 3.

Specific examples of the compound represented by the general formula (1) are shown below. However, the compound is not limited to these exemplified compounds.

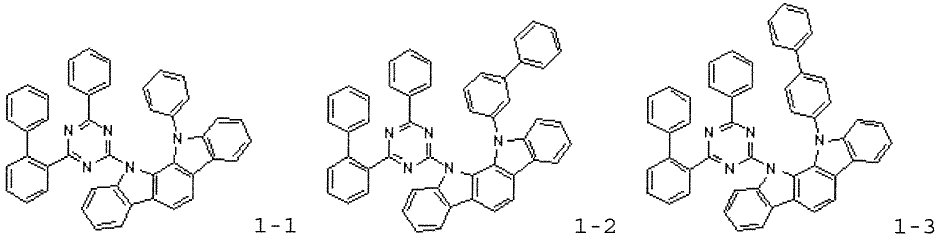

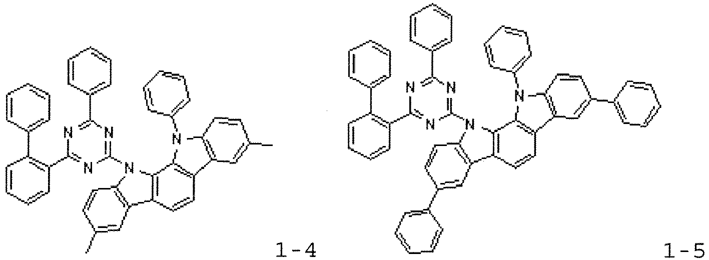

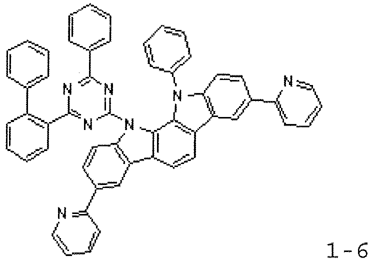

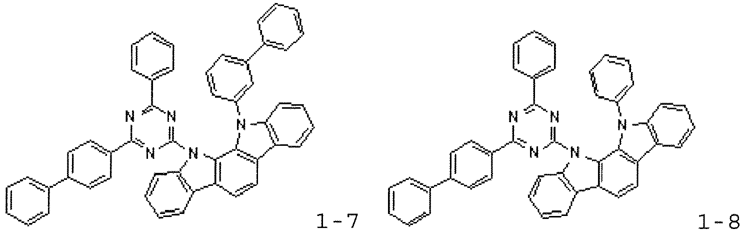

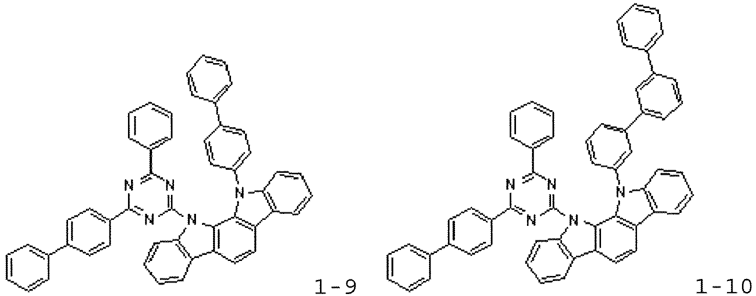

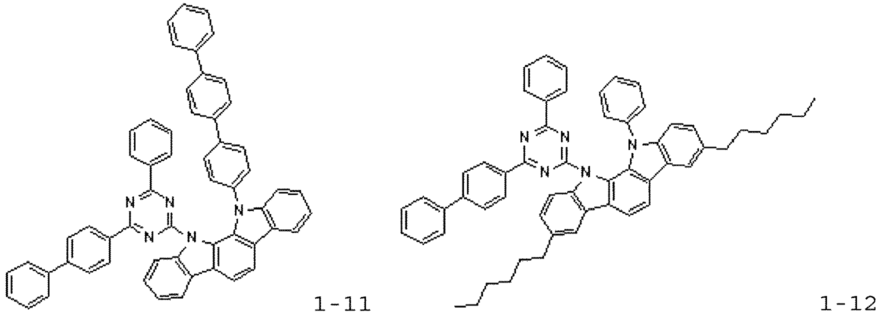

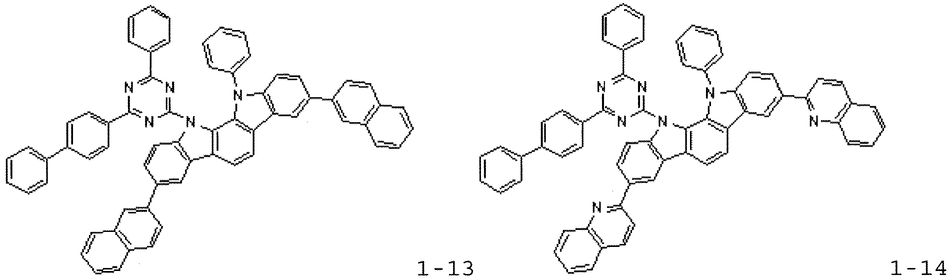

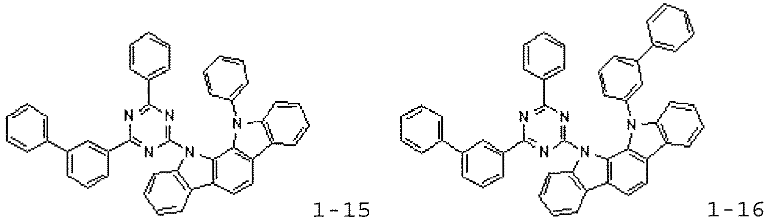

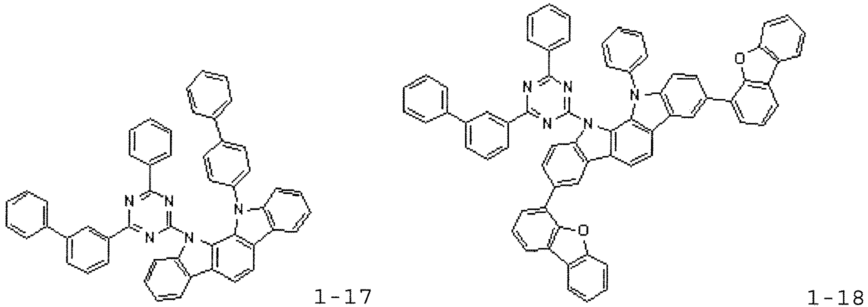

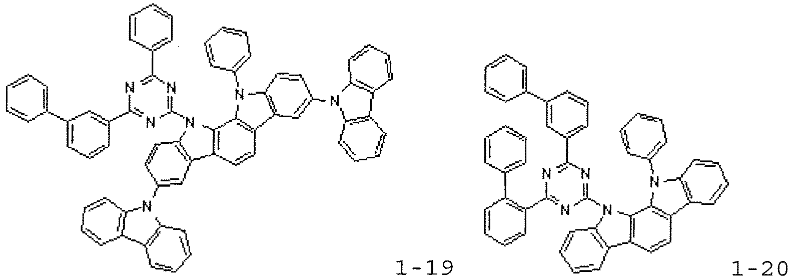

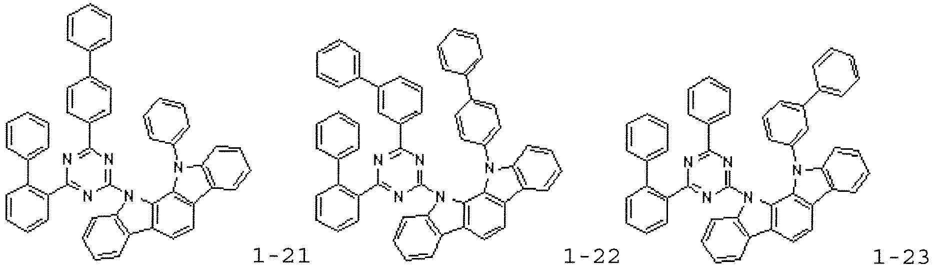

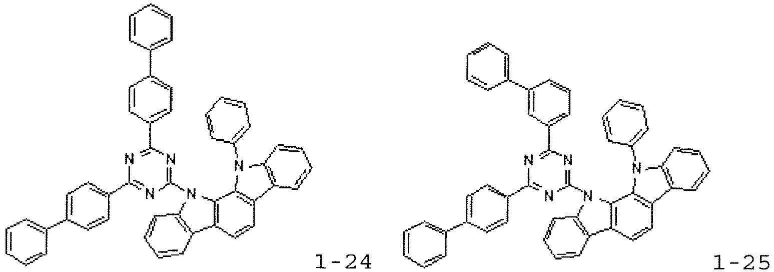

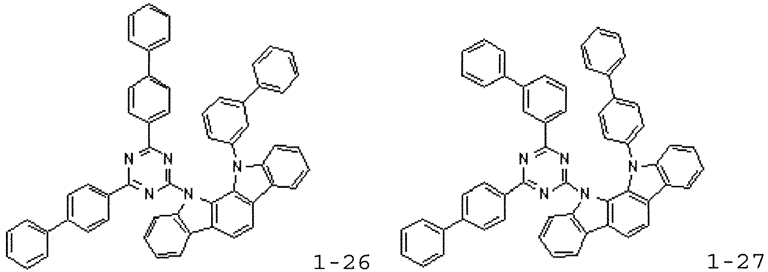

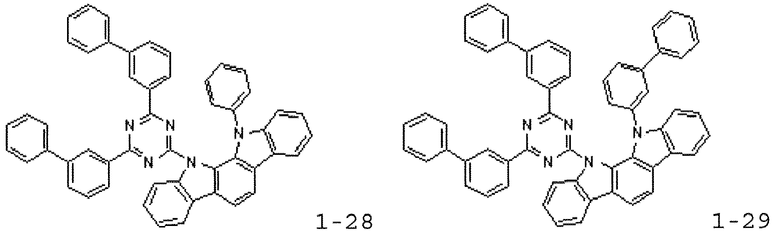

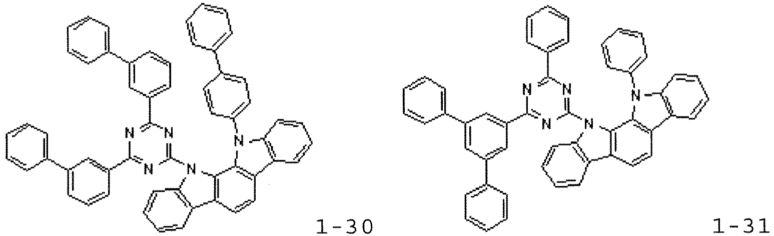

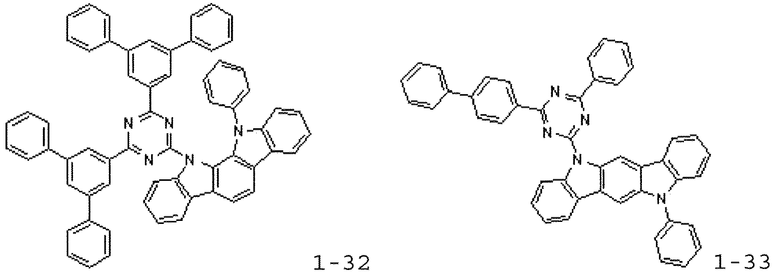

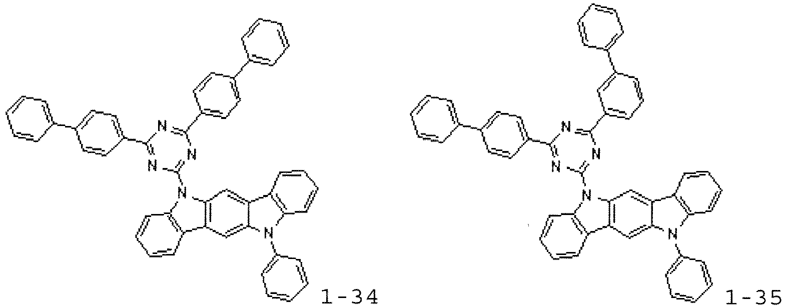

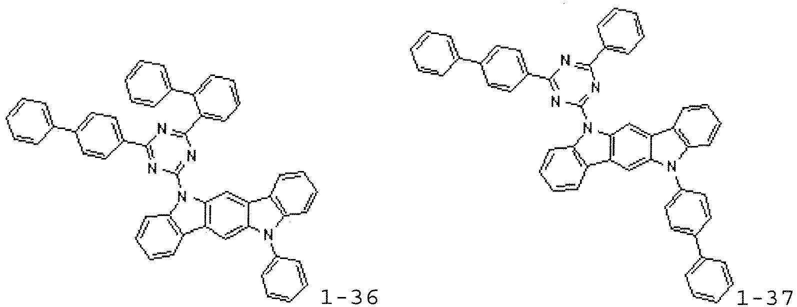

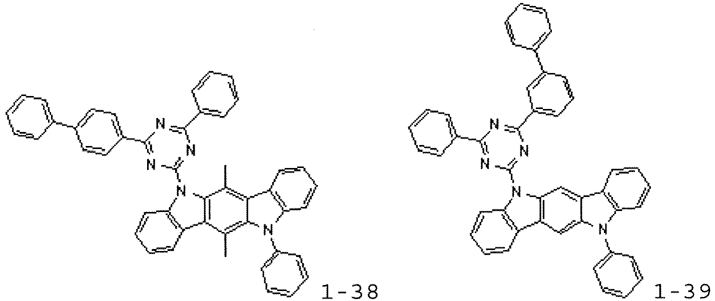

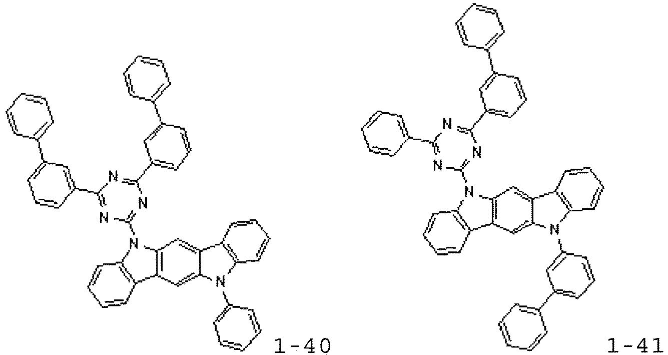

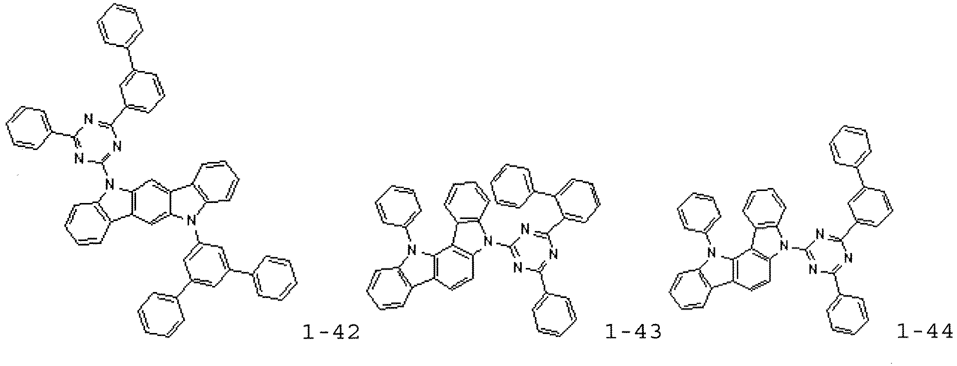

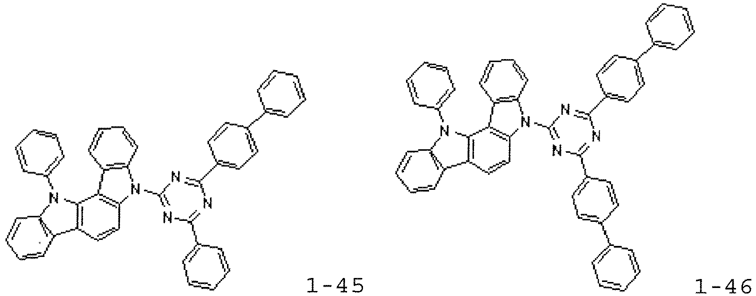

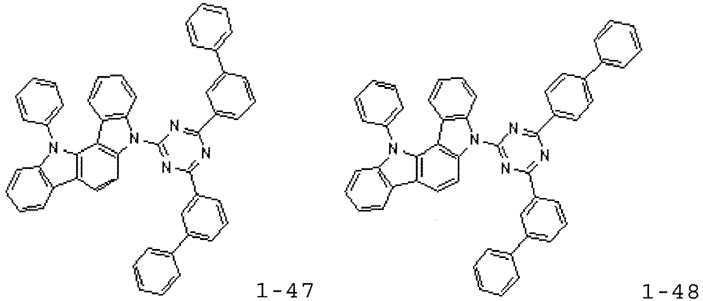

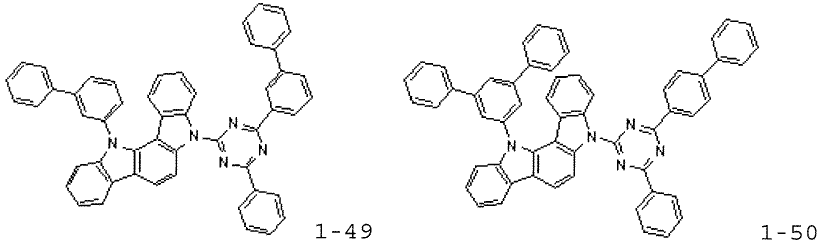

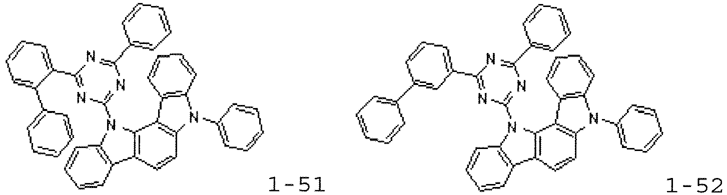

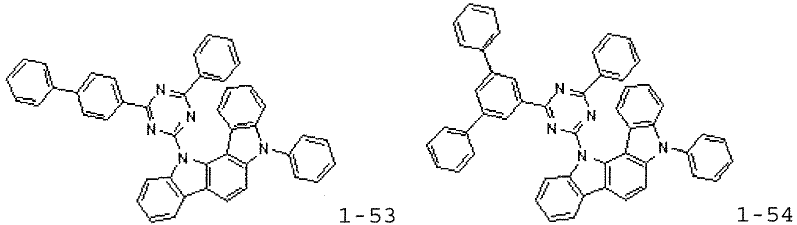

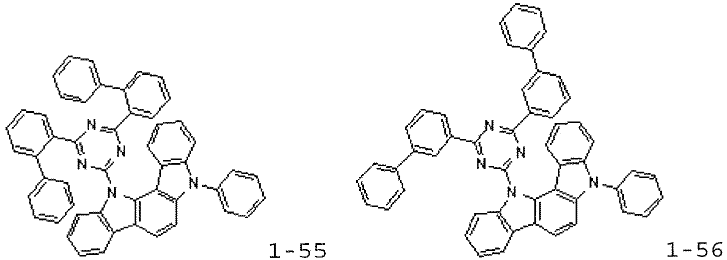

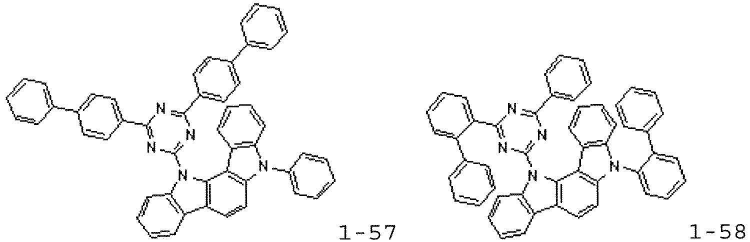

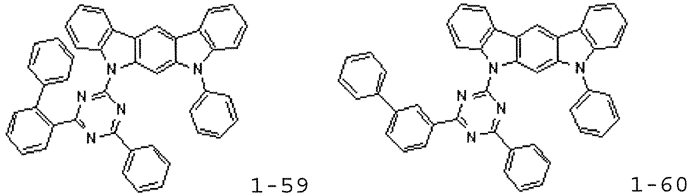

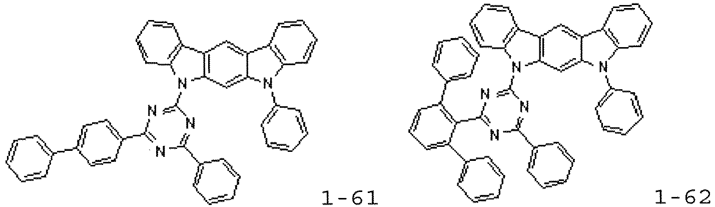

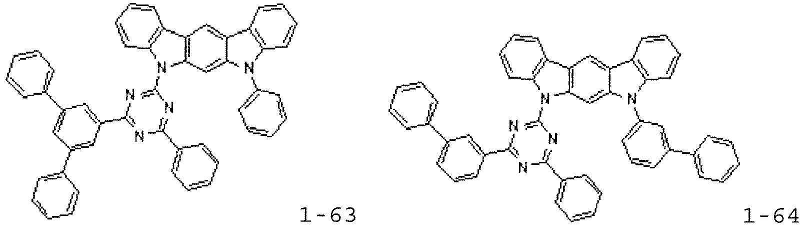

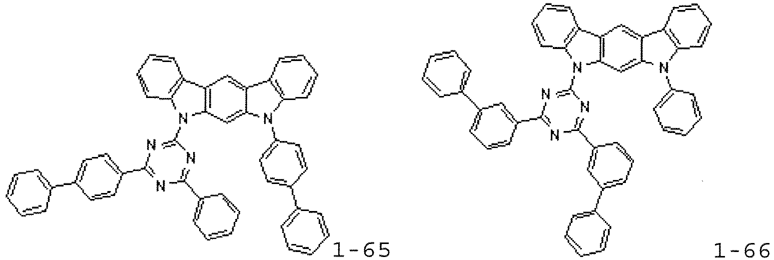

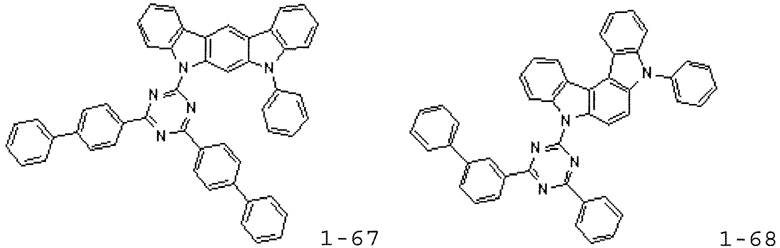

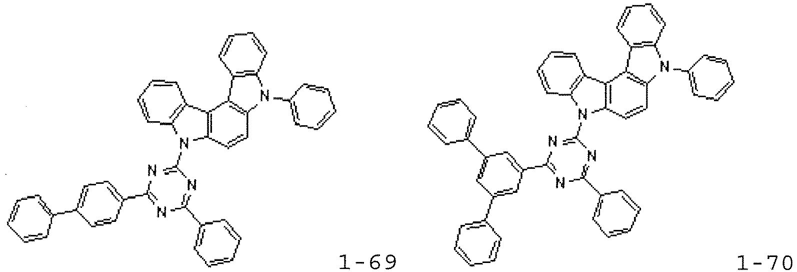

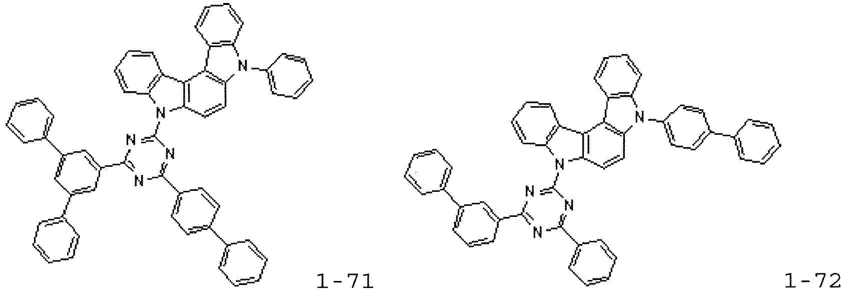

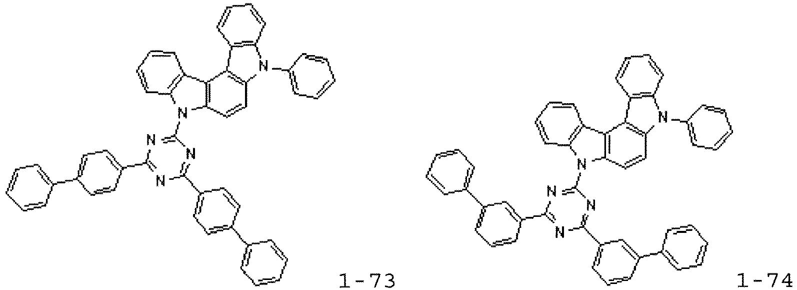

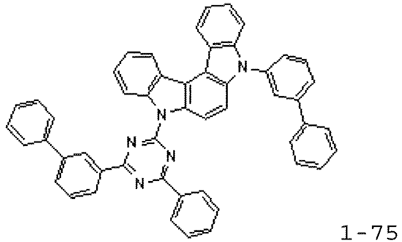

Next, a compound represented by the general formula (2) or the general formula (3) serving as the second host is described. The same symbols in the general formulae (2) and (3) have the same meaning.

Ar² and Ar³ each represent an aromatic hydrocarbon group having 6 to 14 carbon atoms, or a group obtained by linking two or three of the aromatic hydrocarbon groups. Ar² and Ar³ each represent preferably an aromatic hydrocarbon group having 6 to 12 carbon atoms, more preferably an aromatic hydrocarbon group having 6 to 10 carbon atoms, and at least one of Ar² or Ar³ represents a fused aromatic hydrocarbon group.

Specific examples of Ar² and Ar³ include linked aromatic groups each produced by removing one H atom from benzene, naphthalene, anthracene, phenanthrene, fluorene, or a compound produced by linking two or three of the above-mentioned compounds. Preferred examples thereof include aromatic groups each produced from benzene, naphthalene, anthracene, or phenanthrene. More preferred examples thereof include aromatic groups each produced from benzene, naphthalene, or phenanthrene. Ar² still more preferably represents a naphthyl group or a phenanthryl group. Here, the linked aromatic group is a group represented by a formula like -Ar⁵-Ar⁷, -Ar⁵-Ar⁶-Ar⁷, or -Ar⁵ (-Ar⁶) -Ar⁷, and Ar⁵, Ar⁶, and Ar⁷ each independently represent an aromatic hydrocarbon group having 6 to 14 carbon atoms. Ar⁵ represents a divalent or trivalent group, Ar⁶ represents a monovalent or divalent group, and Ar⁷ represents a monovalent group.

Specific examples of the compounds represented by the general formulae (2) and (3) are shown below. However, the compounds are not limited to these exemplified compounds.

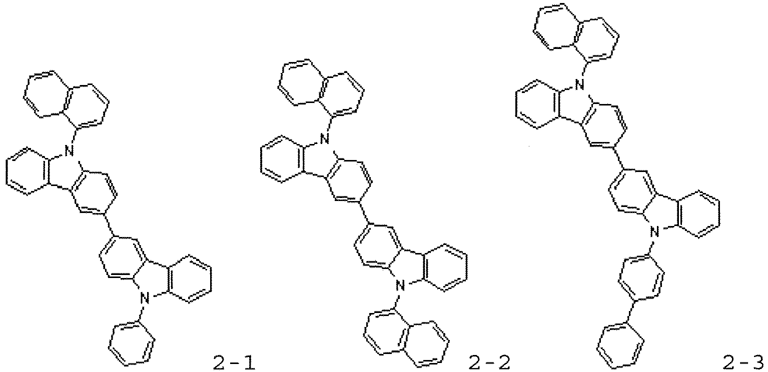

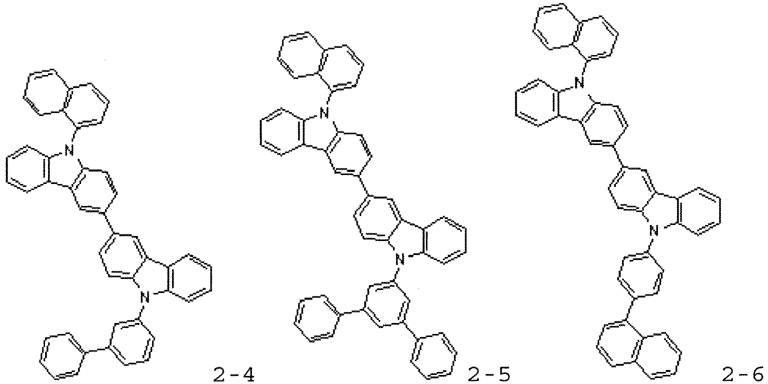

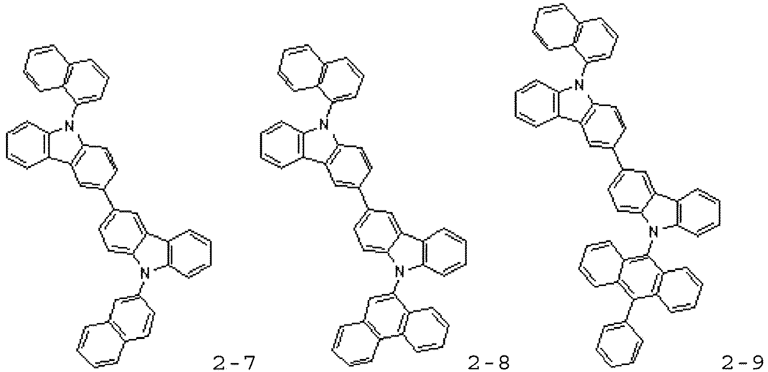

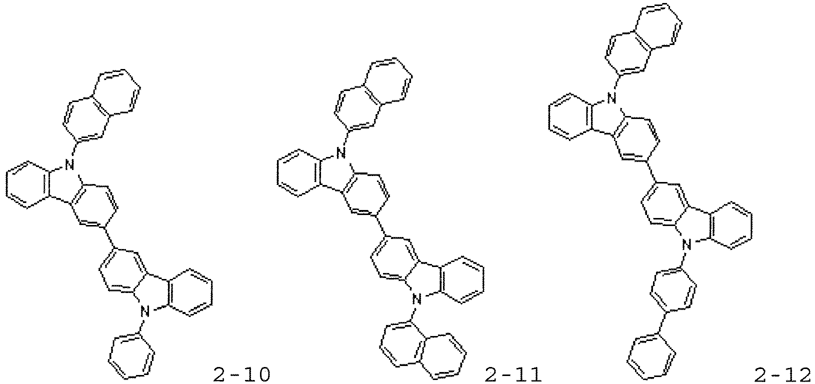

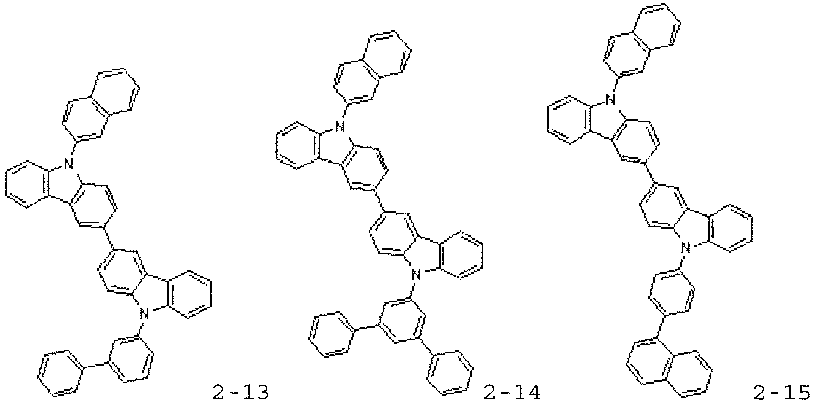

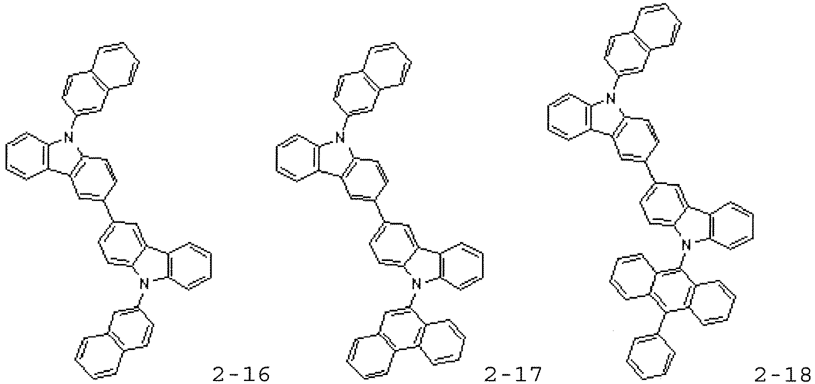

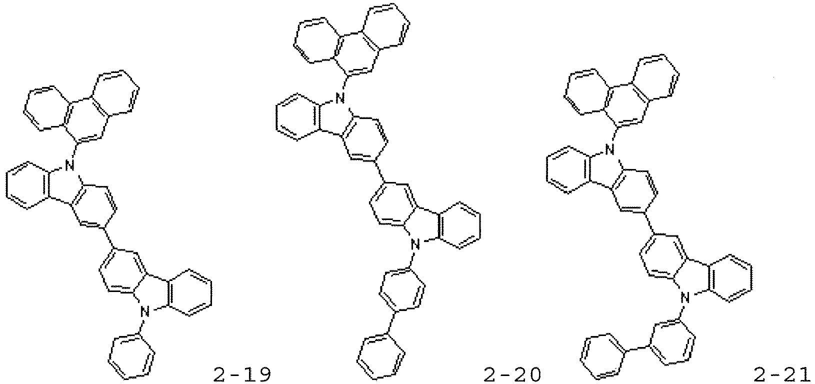

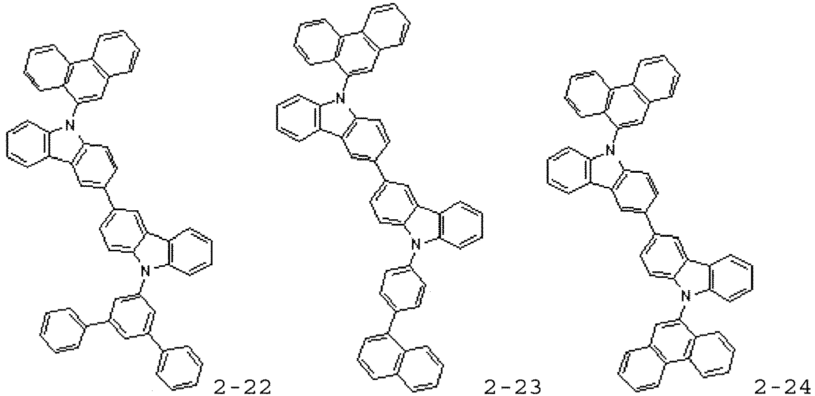

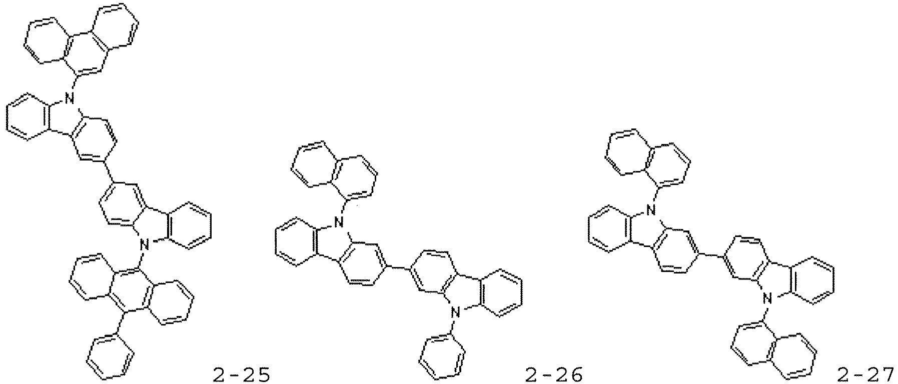

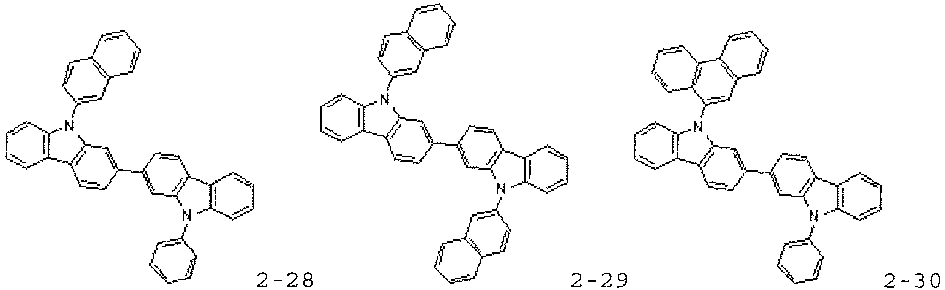

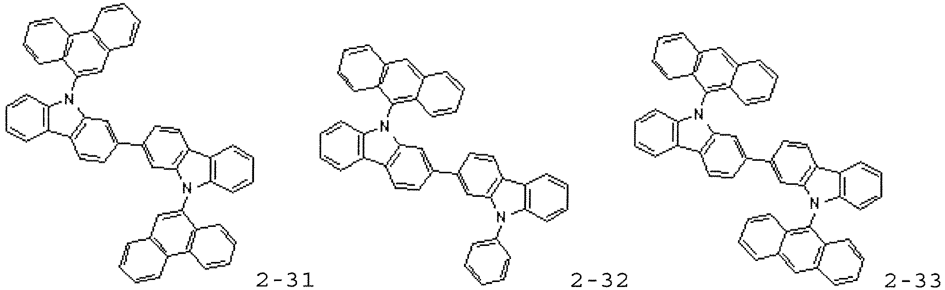

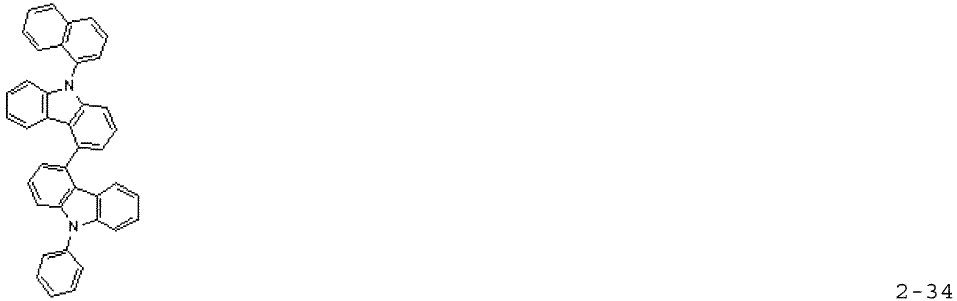

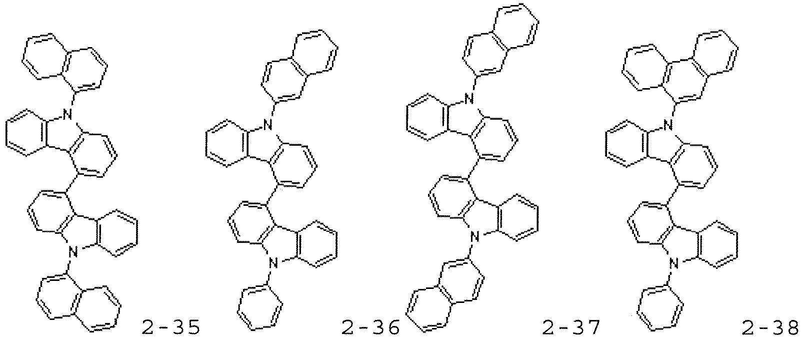

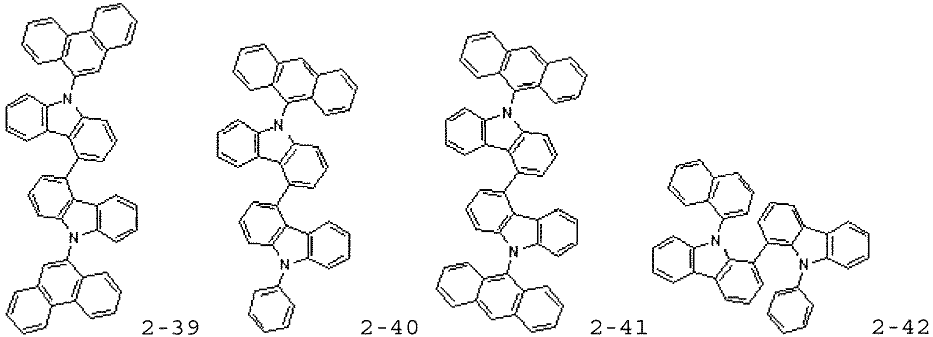

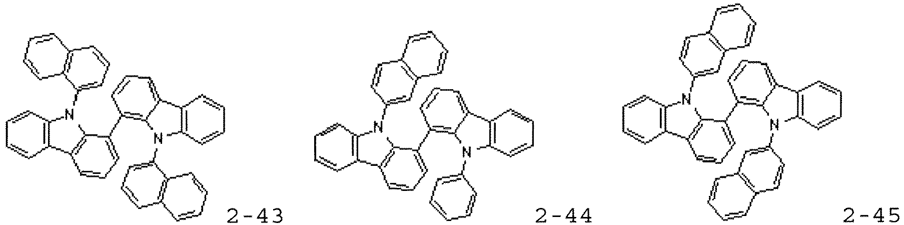

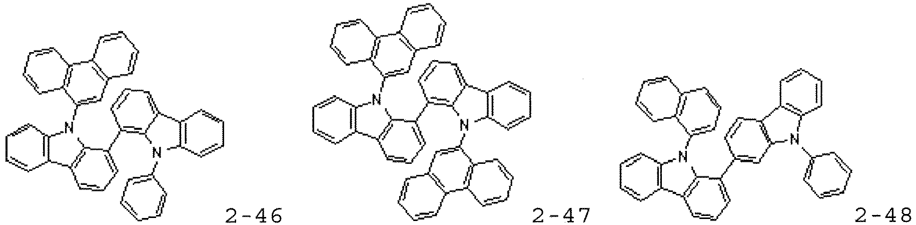

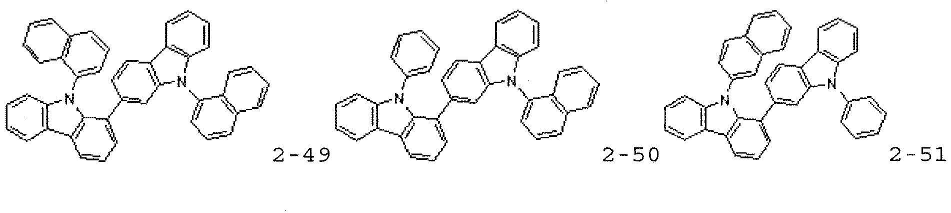

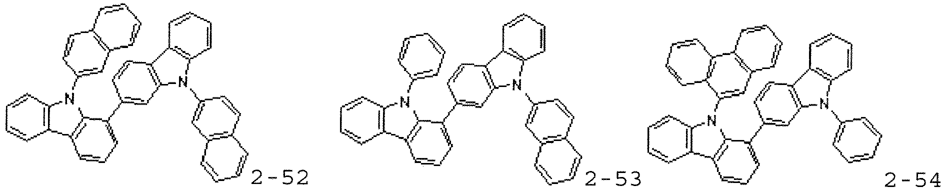

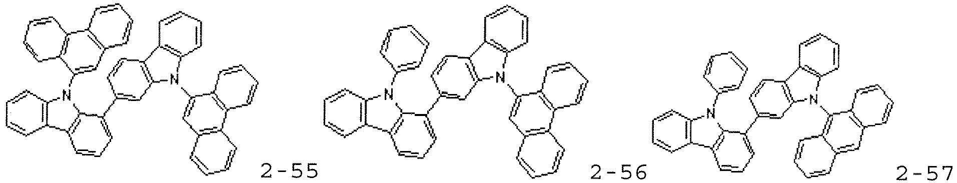

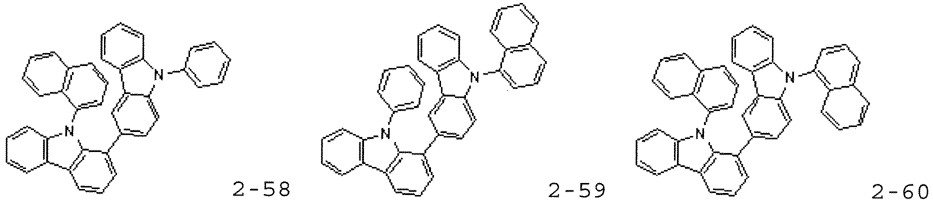

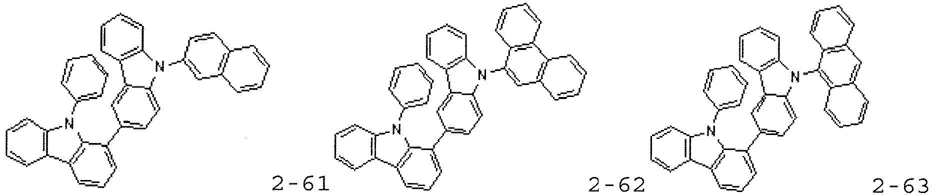

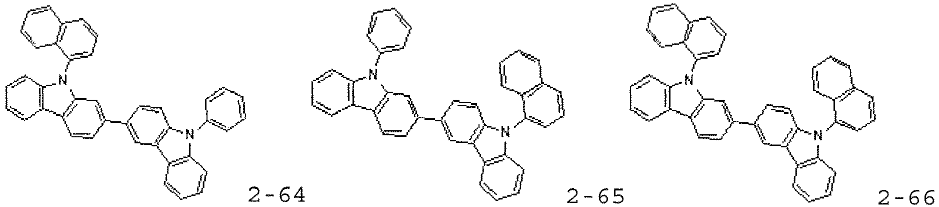

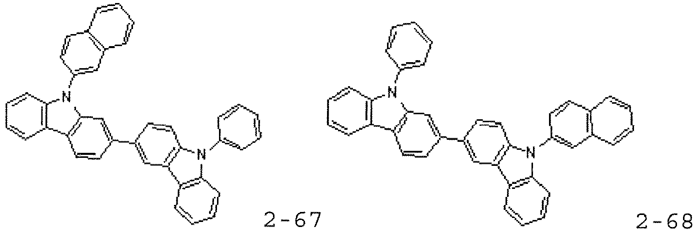

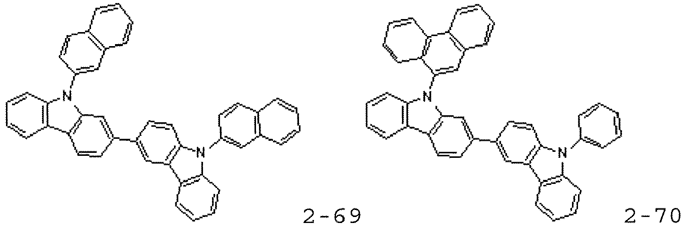

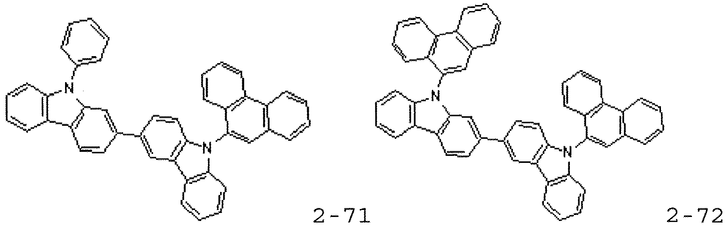

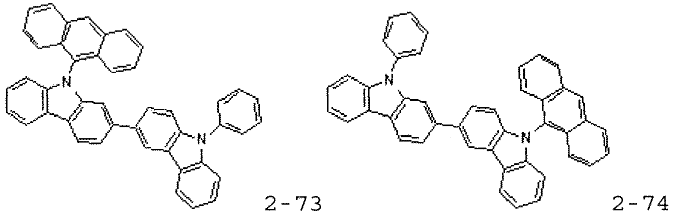

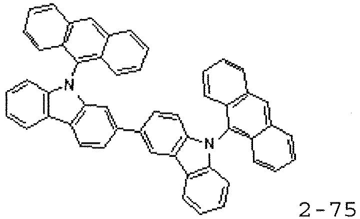

An excellent organic EL device can be provided by using the first host selected from compounds each represented by the general formula (1) and the second host selected from compounds each represented by the general formula (2) as host materials for a light-emitting layer. Although a method of forming the light-emitting layer is not limited, it is advantageous to form the layer by vapor deposition.

When the light-emitting layer is formed by vapor deposition, the first host and the second host can be used by being individually vapor-deposited from different deposition sources, but it is preferred that the hosts be preliminarily mixed before vapor deposition to provide a preliminary mixture and the preliminary mixture be simultaneously vapor-deposited from one deposition source to form the light-emitting layer. In this case, the preliminary mixture may be mixed with the light-emitting dopant material needed for forming the light-emitting layer or any other host to be used as required, but when there is a large difference in temperature at which a desired vapor pressure is obtained between the preliminary mixture and the light-emitting dopant material or the other host, the light-emitting dopant material or the other host is desirably vapor-deposited from another deposition source.

In addition, a mixing ratio (weight ratio) between the first host and the second host is as follows: the ratio of the first host is desirably from 20 wt% to 60 wt%, preferably more than 20 wt% and less than 55 wt%, more preferably from 40 wt% to 50 wt% with respect to the total of the first host and the second host.

In addition, a difference in electron affinity (EA) between the first host and the second host is preferably more than 0.1 eV and less than 0.6 eV. A value for an EA can be calculated by using: a value for the ionization potential (IP) of a thin film of a host material obtained by photoelectron spectroscopy; and a value for an energy gap determined from an absorption edge of an absorption spectrum measured for the thin film.

Next, the structure of the organic EL device of the present invention is described with reference to the drawings. However, the structure of the organic EL device of the present invention is not limited thereto.

FIG. 1 is a sectional view for illustrating a structure example of a general organic EL device used in the present invention. Reference numeral 1 represents a substrate, reference numeral 2 represents an anode, reference numeral 3 represents a hole-injecting layer, reference numeral 4 represents a hole-transporting layer, reference numeral 5 represents a light-emitting layer, reference numeral 6 represents an electron-transporting layer, and reference numeral 7 represents a cathode . The organic EL device of the present invention may include an exciton-blocking layer adjacent to the light-emitting layer, or may include an electron-blocking layer between the light-emitting layer and the hole-injecting layer. The exciton-blocking layer may be inserted on any of the cathode side and the cathode side of the light-emitting layer, and may also be inserted simultaneously on both sides. The organic EL device of the present invention includes the anode, the light-emitting layer, and the cathode as its essential layers. The organic EL device of the present invention preferably includes a hole-injecting/transporting layer and an electron-injecting/transporting layer in addition to the essential layers, and more preferably includes a hole-blocking layer between the light-emitting layer and the electron-injecting/transporting layer. The hole-injecting/transporting layer means any one or both of the hole-injecting layer and the hole-transporting layer, and the electron-injecting/transporting layer means any one or both of an electron-injecting layer and the electron-transporting layer.

It is possible to adopt a reverse structure as compared to FIG. 1, that is, the reverse structure being formed by laminating the layers on the substrate 1 in the order of the cathode 7, the electron-transporting layer 6, the light-emitting layer 5, the hole-transporting layer 4, and the anode 2. In this case as well, some layers may be added or eliminated if necessary.

### -Substrate-

The organic EL device of the present invention is preferably supported by a substrate. The substrate is not particularly limited, and any substrate that has been conventionally used for an organic EL device may be used. For example, a substrate made of glass, a transparent plastic, quartz, or the like may be used.

### -Anode-

A material formed of a metal, an alloy, an electrically conductive compound, or a mixture thereof, which has a large work function (4 eV or more), is preferably used as an anode material in the organic EL device. Specific examples of such electrode material include metals, such as Au, and conductive transparent materials, such as CuI, indium tin oxide (ITO), SnO₂, andZnO. Further, it may be possible to use an amorphous material, such as IDIXO (In₂O₃-ZnO), which may be used for manufacturing a transparent conductive film. In order to produce the anode, it may be possible to form any of those electrode materials into a thin film by using a method such as vapor deposition or sputtering and form a pattern having a desired shape thereon by photolithography. Alternatively, in the case of not requiring high pattern accuracy (about 100 µm or more), a pattern may be formed via a mask having a desired shape when any of the above-mentioned electrode materials is subjected to vapor deposition or sputtering. Alternatively, when a coatable substance, such as an organic conductive compound, is used, it is also possible to use a wet film-forming method, such as a printing method or a coating method. When luminescence is taken out from the anode, the transmittance of the anode is desirably controlled to more than 10%. Further, the sheet resistance as the anode is preferably several hundred Ω/□ or less. The thickness of the film is, depending on its material, selected from usually the range of from 10 nm to 1,000 nm, preferably the range of from 10 nm to 200 nm.

### -Cathode-

Meanwhile, a material formed of a metal (referred to as electron-injecting metal), an alloy, an electrically conductive compound, or a mixture thereof, which has a small work function (4 eV or less), is used as a cathode material. Specific examples of such electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Of those, for example, a mixture of an electron- injecting metal and a second metal as a stable metal having a larger work function value than that of the former metal, such as a magnesium/silver mixture, a magnesium/aluminum mixture, a magnesium/indium mixture, an aluminum/aluminum oxide (Al₂O₃) mixture, or a lithium/aluminum mixture, or aluminum is suitable from the viewpoints of an electron-injecting property and durability against oxidation or the like. The cathode may be produced by forming any of those cathode materials into a thin film by using a method such as vapor deposition or sputtering. Further, the sheet resistance as the cathode is preferably several hundred Ω/□ or less, and the thickness of the film is selected fromusually the range of from 10 nm to 5 µm, preferably the range of from 50 nm to 200 nm. Any one of the anode and cathode of the organic EL device is preferably transparent or semi-transparent because emitted light is transmitted therethrough and the light emission luminance improves.

Further, after any of the above-mentioned metals is formed into a film having a thickness of from 1 nm to 20 nm as a cathode, any of the conductive transparent materials mentioned in the description of the anode is formed into a film on the cathode, thereby being able to produce a transparent or semi-transparent cathode. Then, by applying this, it is possible to produce a device in which both the anode and cathode have transparency.

### -Light-emitting Layer-

The light-emitting layer is a layer that emits light after the production of an exciton by the recombination of a hole injected from the anode and an electron injected from the cathode, and the light-emitting layer contains an organic light-emitting dopant material and a host material.

The first host represented by the general formula (1) and the second host represented by the general formula (2) are used as the host materials in the light-emitting layer. Further, one or more kinds of known host materials may be used in combination with the hosts, but the usage amount thereof is desirably set to 50 wt% or less, preferably 35 wt% or less with respect to the total of the host materials.

The first host and the second host can be respectively vapor-deposited from different deposition sources, or the first host and the second host can be simultaneously vapor-deposited from one deposition source by being preliminarily mixed before the vapor deposition to provide a preliminary mixture.

When the first host and the second host are preliminarily mixed before use, a difference in 50% weight reduction temperature (T₅₀) between the hosts is desirably as small as possible in order that an organic EL device having satisfactory characteristics may be produced with satisfactory reproducibility. The term "50% weight reduction temperature" refers to the temperature at which the weight of a host reduces by 50% when its temperature is increased from room temperature to 550 °C at a rate of 10 °C/min in TG-DTA measurement in a stream of nitrogen under reduced pressure (50 Pa). Vaporization by evaporation or sublimation is considered to occur most vigorously around the temperature.

The difference in 50% weight reduction temperature between the first host and the second host is preferably 20°C or less, more preferably 15°C or less. A known method, such as pulverization mixing, may be adopted as a method for the preliminary mixing, but it is desired that the compounds be mixed as uniformly as possible.

When a phosphorescent light-emitting dopant is used as the light-emitting dopant material, the phosphorescent light-emitting dopant is preferably a phosphorescent light-emitting dopant containing an organometallic complex containing at least one metal selected from ruthenium, rhodium, palladium, silver, rhenium, osmium, iridium, platinum, and gold. Specifically, an iridium complex described in J. Am. Chem. Soc. 2001, 123, 4304 or JP 2013-53051 A is suitably used, but the organometallic complex is not limited thereto.

Only one kind of phosphorescent light-emitting dopant material may be incorporated into the light-emitting layer, or two or more kinds of phosphorescent light-emitting dopant materials maybe incorporated into the layer. The content of the phosphorescent light-emitting dopant material is preferably from 0.1 wt% to 30 wt%, more preferably from 1 wt% to 20 wt% with respect to the host material.

The phosphorescent light-emitting dopant material is not particularly limited, but specific examples thereof include the following materials.

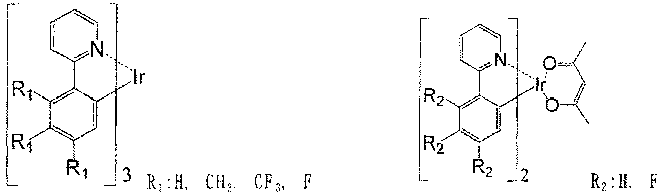

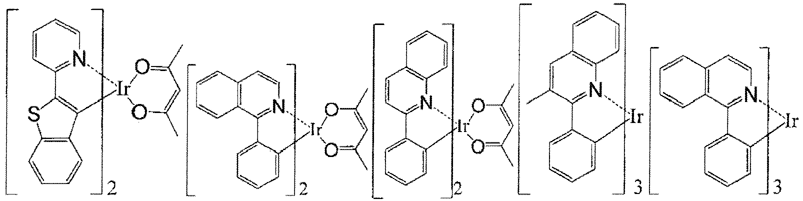

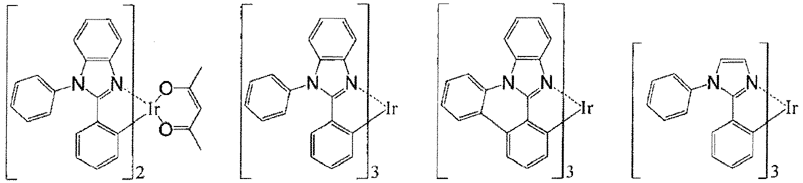

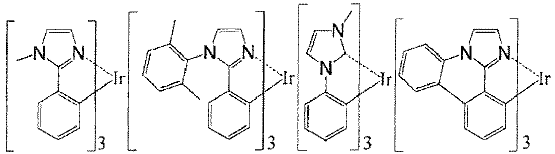

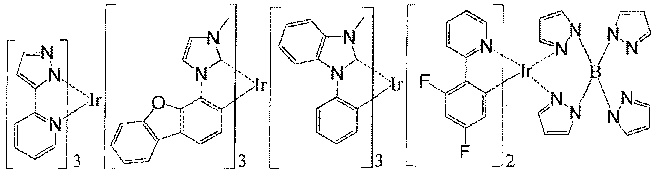

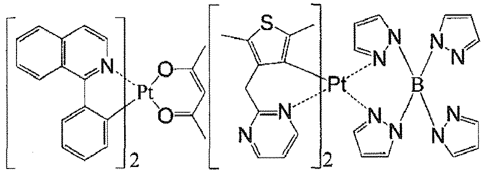

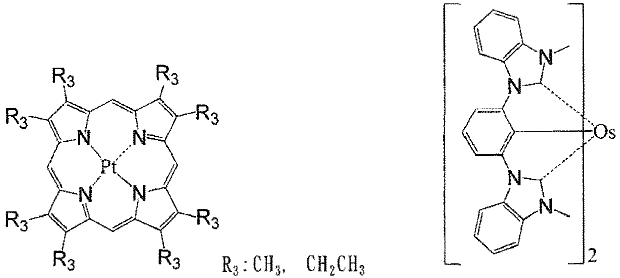

When a fluorescent light-emitting dopant is used as the light-emitting dopant material, the fluorescent light-emitting dopant is not particularly limited, and examples thereof include a benzoxazole derivative, a benzothiazole derivative, a benzimidazole derivative, a styrylbenzene derivative, a polyphenyl derivative, a diphenylbutadiene derivative, a tetraphenylbutadiene derivative, a naphthalimide derivative, a coumarine derivative, a fused aromatic compound, a perinone derivative, an oxadiazole derivative, an oxazine derivative, an aldazine derivative, a pyrrolidine derivative, a cyclopentadiene derivative, a bisstyrylanthracene derivative, a quinacridone derivative, a pyrrolopyridine derivative, a thiadiazolopyridine derivative, a styrylamine derivative, a diketopyrrolopyrrole derivative, an aromatic dimethylidyne compound, various metal complexes typified by a metal complex of an 8 -quinolinol derivative, and a metal complex, rare earth complex, or transition metal complex of a pyrromethene derivative, polymer compounds, such as polythiophene, polyphenylene, and polyphenylene vinylene, and an organic silane derivative. Of those, for example, the following compound is preferred: a fused aromatic derivative, a styryl derivative, a diketopyrrolopyrrole derivative, an oxazine derivative, a pyrromethene metal complex, a transition metal complex, or a lanthanoid complex. For example, the following compound is more preferred: naphthalene, pyrene, chrysene, triphenylene, benzo [c] phenanthrene, benzo [a] anthracene, pentacene, perylene, fluoranthene, acenaphthofluoranthene, dibenzo[a,j]anthracene, dibenzo [a,h] anthracene, benzo[a]naphthalene, hexacene, naphtho[2,1-f]isoquinoline, α-naphthaphenanthridine, phenanthroxazole, quinolino[6,5-f]quinoline, or benzothiophanthrene. Those compounds may each have an alkyl group, an aryl group, an aromatic heterocyclic group, or a diarylamino group as a substituent.

Only one kind of fluorescent light-emitting dopant material may be incorporated into the light-emitting layer, or two or more kinds of fluorescent light-emitting dopant materials may be incorporated into the layer. The content of the fluorescent light-emitting dopant material is preferably from 0.1% to 20%, more preferably from 1% to 10% with respect to the host material.

When a thermally activated delayed fluorescent light-emitting dopant is used as the light-emitting dopant material, the thermally activated delayed fluorescent light-emitting dopant is not particularly limited, and examples thereof include: metal complexes, such as a tin complex and a copper complex; indolocarbazole derivatives described in WO 2011/070963 A; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8, 326.

The thermally activated delayed fluorescent light-emitting dopant material is not particularly limited, but specific examples thereof include the following materials.

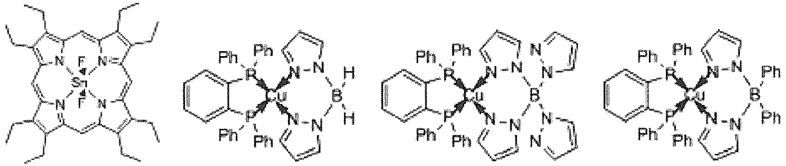

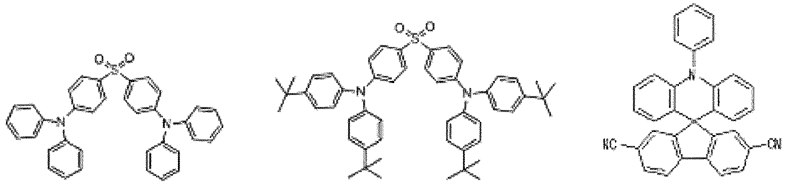

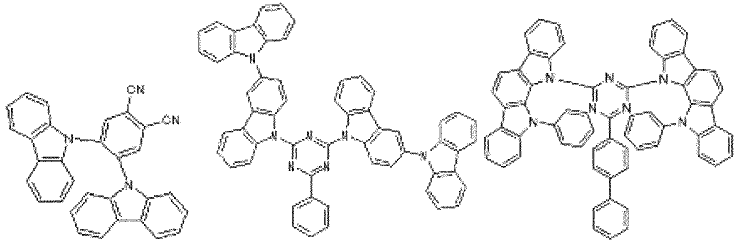

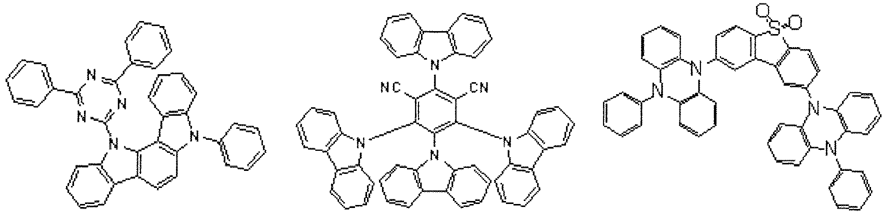

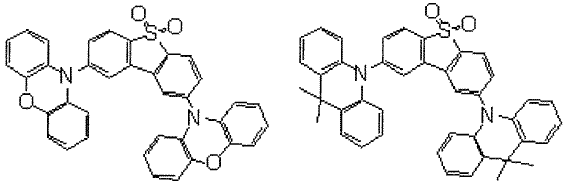

Only one kind of thermally activated delayed fluorescent light-emitting dopant material may be incorporated into the light-emitting layer, or two or more kinds of thermally activated delayed fluorescent light-emitting dopant materials may be incorporated into the layer. In addition, the thermally activated delayed fluorescent light-emitting dopant may be used after having been mixed with a phosphorescent light-emitting dopant or a fluorescent light-emitting dopant. The content of the thermally activated delayed fluorescent light-emitting dopant material is preferably from 0.1% to 50%, more preferably from 1% to 30% with respect to the host material.

### -Injecting Layer-

The injecting layer refers to a layer formed between an electrode and an organic layer for the purposes of lowering a driving voltage and improving light emission luminance, and includes a hole-injecting layer and an electron-injecting layer. The injecting layer may be interposed between the anode and the light-emitting layer or the hole-transporting layer, or may be interposed between the cathode and the light-emitting layer or the electron-transporting layer. The injecting layer may be formed as required.

### -Hole-blocking Layer-

The hole-blocking layer has, in a broad sense, the function of an electron-transporting layer, and is formed of a hole-blocking material that has a remarkably small ability to transport holes while having a function of transporting electrons, and hence the hole-blocking layer is capable of improving the probability of recombining an electron and a hole in the light-emitting layer by blocking holes while transporting electrons.

A compound represented by the general formula (1) is preferably incorporated into the hole-blocking layer, but a known hole-blocking layer material can also be used.

### -Electron-blocking Layer-

The electron-blocking layer has, in a broad sense, the function of a hole-transporting layer, and is capable of improving the probability of recombining an electron and a hole in the light-emitting layer by blocking electrons while transporting holes .

A known material for an electron-blocking layer may be used as a material for the electron-blocking layer, and a material for the hole-transporting layer to be described later may be used as required. The thickness of the electron-blocking layer is preferably from 3 nm to 100 nm, more preferably from 5 nm to 30 nm.

### -Exciton-blocking Layer-

The exciton-blocking layer refers to a layer for blocking excitons produced by the recombination of a hole and an electron in the light-emitting layer from diffusing into charge-transporting layers. The insertion of this layer enables efficient confinement of the excitons in the light-emitting layer, thereby being able to improve the luminous efficiency of the device. In a device in which two or more light-emitting layers are adjacent to each other, the exciton-blocking layer can be inserted between two adjacent light-emitting layers.

A known material for an exciton-blocking layer may be used as a material for the exciton-blocking layer. Examples thereof include 1,3-dicarbazolylbenzene (mCP) and bis(2-methyl-8-quinolinolato)-4-phenylphenolatoaluminum(III) (BAlq) .

### -Hole-transporting Layer-

The hole-transporting layer is formed of a hole-transporting material having a function of transporting holes, and a single hole-transporting layer or a plurality of hole-transporting layers may be formed.

The hole-transporting material has a hole-injecting property or a hole-transporting property or has an electron-blocking property, and any of an organic material and an inorganic material maybe used as the hole-transporting material. Any compound selected from conventionally known compounds may be used for the hole-transporting layer. Examples of such hole-transporting material include a porphyrin derivative, an arylamine derivative, a triazole derivative, an oxadiazole derivative, an imidazole derivative, a polyarylalkane derivative, a pyrazoline derivative, and a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline-based copolymer, and a conductive high-molecular weight oligomer, in particular, a thiophene oligomer. Of those, a porphyrin derivative, an arylamine derivative, or a styrylamine derivative is preferably used, and an arylamine compound is more preferably used.

### -Electron-transporting Layer-

The electron-transporting layer is formed of a material having a function of transporting electrons, and a single electron-transporting layer or aplurality of electron-transporting layers may be formed.

An electron-transporting material (which also serves as a hole-blocking material in some cases) only needs to have a function of transferring electrons injected from the cathode into the light-emitting layer. Any compound selected from conventionally known compounds may be used for the electron-transporting layer. Examples thereof include a polycyclic aromatic derivative, such as naphthalene, anthracene, or phenanthroline, a tris(8-quinolinolato)aluminum(III) derivative, a phosphine oxide derivative, a nitro-substituted fluorene derivative, a diphenylquinone derivative, a thiopyran dioxide derivative, a carbodiimide, a fluorenylidenemethane derivative, anthraquinodimethane and anthrone derivatives, a bipyridine derivative, a quinoline derivative, an oxadiazole derivative, a benzimidazole derivative, a benzothiazole derivative, and an indolocarbazole derivative. Further, it is also possible to use a polymer material in which any of those materials is introduced in a polymer chain or is used as a polymer main chain.

### Examples

The present invention is hereinafter described in more detail by way of Examples. The present invention is not limited to Examples below and may be carried out in various forms as long as the various forms do not deviate from the gist of the present invention.

Compound 1-8 (0.20 g) and Compound 2-10 (0.80 g) were weighed, and were mixed while being ground in a mortar. Thus, a preliminary mixture H1 was prepared.

Preliminary mixtures H2 to H9 were each similarly prepared by using a first host and a second host shown in Table 2.

The kinds and blending ratios of the first host and the second host are shown in Table 2. Compound numbers correspond to numbers attached to the above exemplified compounds.

Chemical formulae for Compounds A and B used as hosts for comparison are shown below.

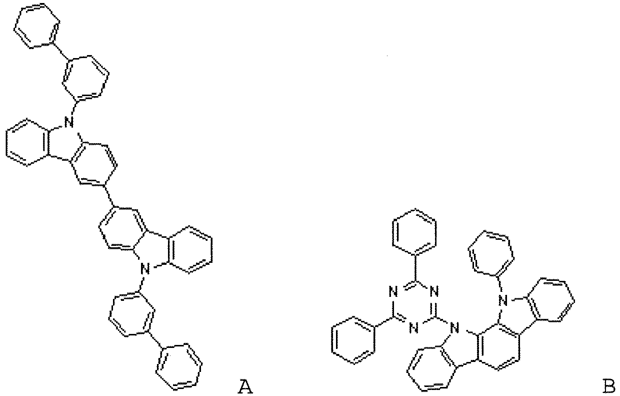

The 50% weight reduction temperatures (T₅₀) and electron affinities (EA) of Compounds 1-8, 1-24, 1-28, 1-46, 1-57, 2-10, 2-16, and 2-19, and Compounds A and B are shown in Table 1.

**Table 1**

| Compound | T₅₀ (°C) | EA (eV) |
|---|---|---|
| 1-8 | 317 | 2.98 |
| 1-24 | 356 | 2.99 |
| 1-28 | 341 | |
| 1-46 | 405 | 2.84 |
| 1-57 | 375 | 2.58 |
| 2-10 | 332 | 2.39 |
| 2-16 | 357 | |
| 2-19 | 348 | |
| A | 363 | |
| B | 281 | 2.68 |

### Example 1

Each thin film was laminated on a glass substrate having formed thereon an anode formed of ITO having a thickness of 110 nm by a vacuum deposition method at a degree of vacuum of 4.0×10⁻⁵ Pa. First, HAT-CN serving as a hole-injecting layer was formed on ITO so as to have a thickness of 25 nm, and then NPD serving as a hole-transporting layer was formed so as to have a thickness of 30 nm. Next, HT-1 serving as an electron-blocking layer was formed so as to have a thickness of 10 nm. Then, the preliminary mixture H1 serving as a host and Ir (ppy)₃ serving as a light-emitting dopant were respectively co-deposited from different deposition sources to form a light-emitting layer having a thickness of 40 nm. At this time, the co-deposition was performed under such a deposition condition that the concentration of Ir (ppy)₃ became 10 wt%. Next, ET-1 serving as an electron-transporting layer was formed so as to have a thickness of 20 nm. Further, lithium fluoride (LiF) serving as an electron-injecting layer was formed on the electron-transporting layer so as to have a thickness of 1 nm. Finally, aluminum (Al) serving as a cathode was formed on the electron-injecting layer so as to have a thickness of 70 nm. Thus, an organic EL device was produced.

### Examples 2 to 9

Organic EL devices were each produced in the same manner as in Example 1 except that in Example 1, any one of the preliminary mixtures H2 to H9 was used as a host.

### Example 10

An organic EL device was produced in the same manner as in Example 3 except that in Example 3, after the formation of the light-emitting layer, Compound 1-8 serving as a hole-blocking layer was formed so as to have a thickness of 10 nm, and ET-1 serving as an electron-transporting layer was formed so as to have a thickness of 10 nm.

### Example 11

Each thin film was laminated on a glass substrate having formed thereon an anode formed of ITO having a thickness of 110 nm by a vacuum deposition method at a degree of vacuum of 4.0×10⁻⁵ Pa. First, HAT-CN serving as a hole-injecting layer was formed on ITO so as to have a thickness of 25 nm, and then NPD serving as a hole-transporting layer was formed so as to have a thickness of 30 nm. Next, HT-1 serving as an electron-blocking layer was formed so as to have a thickness of 10 nm. Next, Compound 1-8 serving as a first host, Compound 2-10 serving as a second host, and Ir(ppy)₃ serving as a light-emitting dopant were respectively co-deposited from different deposition sources to form a light-emitting layer having a thickness of 40 nm. At this time, the co-deposition was performed under such a deposition condition that the concentration of Ir(ppy)₃ became 10 wt% and the weight ratio between the first host and the second host became 40:60. Next, ET-1 serving as an electron-transporting layer was formed so as to have a thickness of 20 nm. Further, LiF serving as an electron-injecting layer was formed on the electron-transporting layer so as to have a thickness of 1 nm. Finally, Al serving as a cathode was formed on the electron-injecting layer so as to have a thickness of 70 nm. Thus, an organic EL device was produced.

### Example 12

An organic EL device was produced in the same manner as in Example 11 except that in Example 11, Compound 1-8 was used as the first host and Compound 2-19 was used as the second host.

### Example 13

An organic EL device was produced in the same manner as in Example 11 except that in Example 11, Compound 1-24 was used as the first host and Compound 2-16 was used as the second host.

### Example 14

An organic EL device was produced in the same manner as in Example 11 except that in Example 11, Compound 1-46 was used as the first host and Compound 2-19 was used as the second host.

### Example 15

Each thin film was laminated on a glass substrate having formed thereon an anode formed of ITO having a thickness of 110 nm by a vacuum deposition method at a degree of vacuum of 4.0×10⁻⁵ Pa. First, HAT-CN serving as a hole-injecting layer was formed on ITO so as to have a thickness of 25 nm, and then NPD serving as a hole-transporting layer was formed so as to have a thickness of 45 nm. Next, HT-1 serving as an electron-blocking layer was formed so as to have a thickness of 10 nm. Then, the preliminary mixture H2 serving as a host and Ir(piq)₂acac serving as a light-emitting dopant were respectively co-deposited from different deposition sources to form a light-emitting layer having a thickness of 40 nm. At this time, the co-deposition was performed under such a deposition condition that the concentration of Ir(piq)₂acac became 6.0 wt%. Further, Compound 1-8 serving as a hole-blocking layer was formed so as to have a thickness of 10 nm. Next, ET-1 serving as an electron-transporting layer was formed so as to have a thickness of 27.5 nm. Then, LiF serving as an electron-injecting layer was formed on the electron-transporting layer so as to have a thickness of 1 nm. Finally, Al serving as a cathode was formed on the electron-injecting layer so as to have a thickness of 70 nm. Thus, an organic EL device was produced.

### Examples 16 and 17

Organic EL devices were each produced in the same manner as in Example 15 except that in Example 15, any one of the preliminary mixtures H3 and H4 was used as a host.

### Example 18

Each thin film was laminated on a glass substrate having formed thereon an anode formed of ITO having a thickness of 110 nm by a vacuum deposition method at a degree of vacuum of 4.0×10⁻⁵ Pa. First, HAT-CN serving as a hole-injecting layer was formed on ITO so as to have a thickness of 25 nm, and then NPD serving as a hole-transporting layer was formed so as to have a thickness of 45 nm. Next, HT-1 serving as an electron-blocking layer was formed so as to have a thickness of 10 nm. Then, Compound 1-57 serving as a first host, Compound 2-16 serving as a second host, and Ir(piq)2acac serving as a light-emitting dopant were respectively co-deposited from different deposition sources to form a light-emitting layer having a thickness of 40 nm. At this time, the co-deposition was performed under such a deposition condition that the concentration of Ir (piq)₂acac became 6.0 wt% and the weight ratio between the first host and the second host became 30:70. Further, Compound 1-8 serving as a hole-blocking layer was formed so as to have a thickness of 10 nm. Next, ET-1 serving as an electron-transporting layer was formed so as to have a thickness of 27.5 nm. Then, LiF serving as an electron-injecting layer was formed on the electron-transporting layer so as to have a thickness of 1 nm. Finally, Al serving as a cathode was formed on the electron-injecting layer so as to have a thickness of 70 nm. Thus, an organic EL device was produced.

### Example 19

An organic EL device was produced under the same conditions as those of Example 18 except that in Example 18, the co-deposition was performed under such a deposition condition that the weight ratio between the first host and the second host became 40:60.

### Example 20

An organic EL device was produced under the same conditions as those of Example 18 except that in Example 18, the co-deposition was performed under such a deposition condition that the weight ratio between the first host and the second host became 50:50.

### Comparative Example 1

An organic EL device was produced in the same manner as in Example 1 except that in Example 1, Compound 1-8 was used alone as a host. The thickness and light-emitting dopant concentration of its light-emitting layer are the same as those of Example 1.

### Comparative Examples 2 to 7

Organic EL devices were each produced in the same manner as in Example 1 except that in Example 1, a compound shown in Table 2 was used alone as a host.

### Comparative Example 8

An organic EL device was produced in the same manner as in Example 11 except that in Example 11, Compound 1-8 was used as the first host and Compound A was used as the second host.

### Comparative Example 9

An organic EL device was produced in the same manner as in Example 11 except that in Example 11, Compound B was used as the first host and Compound 2-10 was used as the second host.

### Comparative Examples 10 and 11

Organic EL devices were each produced in the same manner as in Example 15 except that in Example 15, Compound 1-8 or Compound 1-57 was used alone as a host.

The compounds used in Examples are shown below.

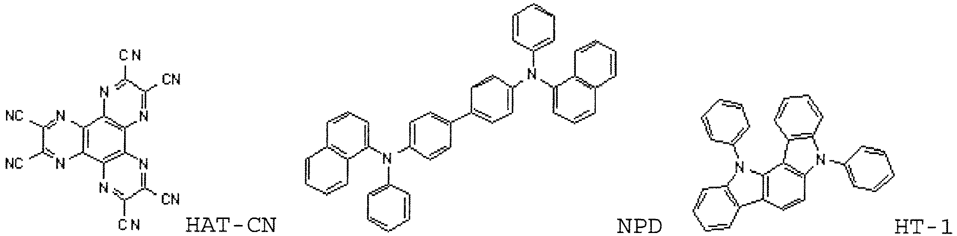

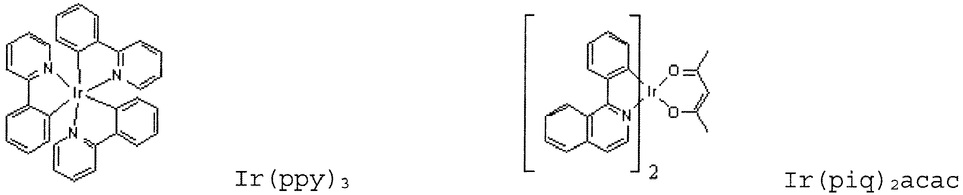

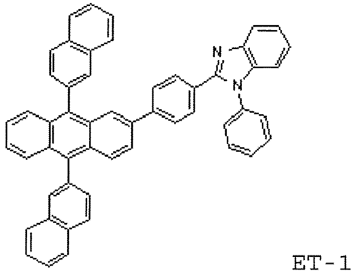

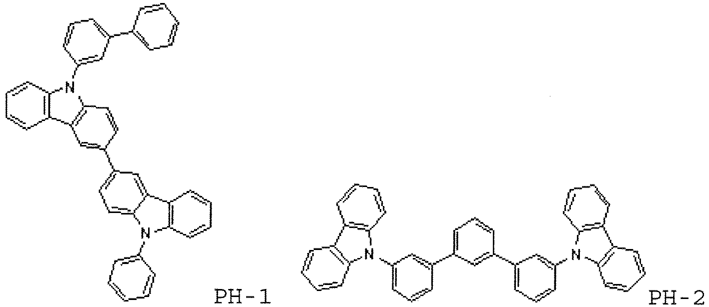

The kinds of the preliminary mixtures each containing the first host and the second host, and the kinds and ratios of the first host and the second host are shown in Tables 2 and 3.

**Table 2**

| Example | Preliminary mixture | First host compound | Second host compound |
|---|---|---|---|
| 1 | H1 | 1-8 (20%) | 2-10 (80%) |
| 2 | H2 | 1-8 (30%) | 2-10 (70%) |
| 3 | H3 | 1-8 (40%) | 2-10 (60%) |
| 4 | H4 | 1-8 (50%) | 2-10 (50%) |
| 5 | H5 | 1-8 (60%) | 2-10 (40%) |
| 6 | H6 | 1-28 (40%) | 2-19 (60%) |
| 7 | H7 | 1-28 (60%) | 2-19 (40%) |
| 8 | H8 | 1-8 (40%) | 2-19 (60%) |
| 9 | H9 | 1-8 (60%) | 2-19 (40%) |
| 10 | H3 | 1-8 (40%) | 2-10 (60%) |
| 11 | - | 1-8 (40%) | 2-10 (60%) |
| 12 | - | 1-8 (40%) | 2-19 (60%) |
| 13 | - | 1-24 (40%) | 2-16 (60%) |
| 14 | - | 1-46 (40%) | 2-19 (60%) |
| 15 | H2 | 1-8 (30%) | 2-10 (70%) |
| 16 | H3 | 1-8 (40%) | 2-10 (60%) |
| 17 | H4 | 1-8 (50%) | 2-10 (50%) |
| 18 | - | 1-57 (30%) | 2-16 (70%) |
| 19 | - | 1-57 (40%) | 2-16 (60%) |
| 20 | - | 1-57 (50%) | 2-16 (50%) |

**Table 3**

| Comparative Example | Preliminary mixture | First host compound | Second host compound |
|---|---|---|---|
| 1 | - | 1-8 | - |
| 2 | - | 1-28 | - |
| 3 | - | 1-24 | - |
| 4 | - | 1-46 | - |
| 5 | - | - | 2-10 |
| 6 | - | - | 2-19 |
| 7 | - | | 2-16 |
| 8 | - | 1-8 (40%) | A (60%) |
| 9 | - | B (40%) | 2-10 (60%) |
| 10 | - | 1-8 | - |
| 11 | - | 1-57 | - |

When an external power source was connected to each of the organic EL devices produced in Examples 1 to 14 and Comparative Examples 1 to 9 to apply a DC voltage to the device, an emission spectrum having a maximum wavelength of 530 nm was observed, and hence it was found that light emission from Ir (ppy)₃ was obtained. In addition, in each of the organic EL devices produced in Examples 15 to 20 and Comparative Examples 10 and 11, an emission spectrum having a maximum wavelength of 620 nm was observed, and hence it was found that light emission from Ir(pic)₂acac was obtained.

The luminances, driving voltages, luminous efficiencies, and luminance half-lives of the produced organic EL devices are shown in Tables 4 and 5. In each of the tables, the luminance, the driving voltage, and the luminous efficiency are values at a driving current of 20 mA/cm², and are initial characteristics. In Table 4, the column "LT70" shows a time required for a luminance to attenuate from an initial luminance of 9,000 cd/m² to 70% of the initial luminance, and in Table 5, the column "LT95" shows a time required for a luminance to attenuate from an initial luminance of 3,700 cd/m² to 95% of the initial luminance. The LT70 and the LT95 are lifetime characteristics.

**Table 4**

| | Luminance (cd/m²) | Voltage (V) | Luminous efficiency (lm/W) | LT70 (h) |
|---|---|---|---|---|
| Example 1 | 8,850 | 4.7 | 29.5 | 800 |
| 2 | 10,560 | 4.3 | 38.6 | 1,030 |
| 3 | 11,500 | 3.9 | 46.3 | 1,120 |
| 4 | 11,700 | 3.7 | 51.0 | 1,050 |
| 5 | 11,870 | 3.6 | 56.5 | 890 |
| 6 | 11,450 | 4.0 | 44.9 | 1,330 |
| 7 | 11,800 | 3.8 | 48.8 | 860 |
| 8 | 11,820 | 3.6 | 51.5 | 880 |
| 9 | 12,110 | 3.5 | 54.3 | 530 |
| 10 | 11,650 | 3.7 | 49.4 | 1,340 |
| 11 | 11,480 | 3.9 | 46.2 | 1,130 |
| 12 | 11,290 | 3.8 | 46.6 | 1,200 |
| 13 | 10,980 | 3.7 | 46.6 | 1,030 |
| 14 | 11,890 | 4.1 | 45.5 | 1,010 |
| Comparative Example 1 | 12,300 | 3.4 | 56.8 | 320 |
| 2 | 13,130 | 3.5 | 58.9 | 370 |
| 3 | 11,110 | 3.2 | 54.5 | 270 |
| 4 | 11,390 | 3.5 | 51.1 | 240 |
| 5 | 1,890 | 7.2 | 4.1 | 30 |
| 6 | 1,970 | 7.0 | 4.4 | 45 |
| 7 | 2,040 | 6.9 | 4.6 | 55 |
| 8 | 11,050 | 4.5 | 38.6 | 410 |
| 9 | 11,230 | 3.8 | 46.4 | 580 |

**Table 5**

| | Luminance (cd/m²) | Voltage (V) | Luminous efficiency (lm/W) | LT95 (h) |
|---|---|---|---|---|
| Example 15 | 3,980 | 4.2 | 14.9 | 380 |
| 16 | 3,770 | 3.9 | 15.2 | 330 |
| 17 | 3,570 | 3.6 | 15.6 | 280 |
| 18 | 3,870 | 4.4 | 13.8 | 340 |
| 19 | 3,800 | 4.3 | 13.9 | 300 |
| 20 | 3,490 | 3.9 | 14.0 | 240 |
| Comparative Example 10 | 2,190 | 2.8 | 12.3 | 20 |
| 11 | 2,380 | 3.2 | 11.7 | 10 |

It is found from Table 4 and Table 5 that in the case where the first host represented by the general formula (1) and the second host represented by the general formula (2) are mixed before use, the lifetime characteristics are significantly extended as compared to those in the case where each of the hosts is used alone. It is also found that even when the first host and the second host are mixed before use, in the case where one of the hosts is not a compound represented by the general formula (1) or the general formula (2), the driving voltage is high and hence satisfactory lifetime characteristics are not obtained.

It is also found that the use of a compound represented by the general formula (1) as a hole-blocking layer material like each of Examples 10 and 15 to 18 extends the lifetime characteristics.

Compound 1-8 (0.40 g), Compound 2-10 (0.30 g), and PH-1 (0.30 g) were weighed, and were mixed while being ground in a mortar. Thus, a preliminary mixture H10 was prepared.

Compound 1-8 (0.40 g), Compound 2-10 (0.30 g), and PH-2 (0.30 g) were weighed, and were mixed while being ground in a mortar. Thus, a preliminary mixture H11 was prepared.

Blending ratios in the preliminary mixtures H10 and H11 were as follows : the preliminary mixtures each contained 40% of the first host (Compound 1-8), 30% of the second host (Compound 2-10), and 30% of the third host (PH-1 or PH-2).

### Example 21

An organic EL device was produced in the same manner as in Example 1 except that in Example 1, the preliminary mixture H10 was used as a host.

### Example 22

An organic EL device was produced in the same manner as in Example 1 except that in Example 1, the preliminary mixture H11 was used as a host.

When an external power source was connected to each of the organic EL devices produced in Examples 21 and 22 to apply a DC voltage to the device, an emission spectrum having a maximum wavelength of 530 nm was observed, and hence it was found that light emission from Ir(ppy)₃ was obtained.

The luminances, driving voltages, luminous efficiencies, and luminance half-lives of the produced organic EL devices are shown in Table 6. In Table 6, the luminance, the driving voltage, and the luminous efficiency are values at a driving current of 20 mA/cm², and are initial characteristics. In Table 6, the column "LT70" shows a time required for a luminance to attenuate from an initial luminance of 9,000 cd/m² to 70% of the initial luminance. The LT70 is a lifetime characteristic.

**Table 6**

| Example | Luminance (cd/m²) | Voltage (V) | Luminous efficiency (lm/W) | LT70 (h) |
|---|---|---|---|---|
| 21 | 12,335 | 3.9 | 49.7 | 1,280 |
| 22 | 12,970 | 4.2 | 48.5 | 1,350 |

### Reference Signs List

1 substrate, 2 anode, 3 hole-injecting layer, 4 hole-transporting layer, 5 light-emitting layer, 6 electron-transporting layer, 7 cathode

## Claims

1. An organic electroluminescent device, comprising one or more light-emitting layers between an anode and a cathode opposite to eachother, whereinat least one of the light-emitting layers contains a first host selected from compounds each represented by the following general formula (1), a second host selected from compounds each represented by the following general formula (2), and a light-emitting dopant material: 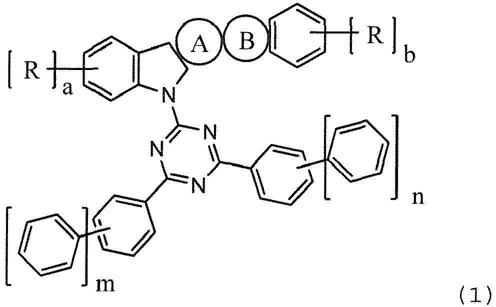 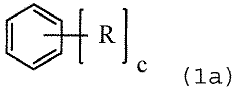 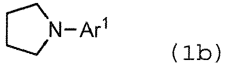 wherein,
a ring A comprises an aromatic hydrocarbon ring represented by the formula (1a), a ring B comprises a heterocycle represented by the formula (1b), and the ring A and the ring B are each fused with an adjacent ring at arbitrary positions;
Ar¹ represents a phenyl group, a biphenyl group, or a terphenyl group;
R's each independently represents an aliphatic hydrocarbon group having 1 to 10 carbon atoms, an aromatic hydrocarbon group having 6 to 10 carbon atoms, or an aromatic heterocyclic group having 3 to 12 carbon atoms;
a, b, and c each independently represent an integer of from 0 to 3; and
m and n each independently represent an integer of from 0 to 2, and m+n represents an integer of 1 or more; 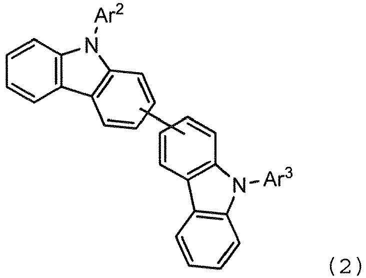
wherein, Ar² and Ar³ each represent an aromatic hydrocarbon group having 6 to 14 carbon atoms, or a group obtained by linking two or three of the aromatic hydrocarbon groups, and at least one of Ar² or Ar³ represents a fused aromatic hydrocarbon group.

2. An organic electroluminescent device according to claim 1, wherein the compound represented by the general formula (2) comprises a compound represented by the following general formula (3): 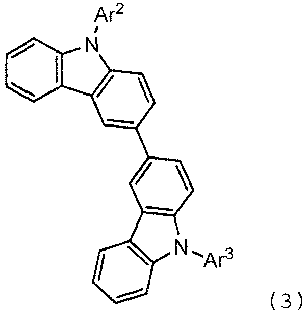 wherein, Ar² and Ar³ are identical in meaning to Ar² and Ar³ of the general formula (2).

3. An organic electroluminescent device according to claim 1, wherein Ar² represents a naphthyl group or a phenanthryl group.

4. An organic electroluminescent device according to claim 1, wherein the organic electroluminescent device comprises a light-emitting layer obtained by vapor-depositing a host material containing a preliminary mixture of the first host and the second host.

5. An organic electroluminescent device according to claim 1, wherein a difference in 50% weight reduction temperature between the first host and the second host is 20°C or less.

6. An organic electroluminescent device according to claim 1, wherein a ratio of the first host is more than 20 wt% and less than 55 wt% with respect to a total of the first host and the second host.

7. An organic electroluminescent device according to any one of claims 1 to 6, wherein the light-emitting dopant material comprises an organometallic complex containing at least one metal selected from the group consisting of ruthenium, rhodium, palladium, silver, rhenium, osmium, iridium, platinum, and gold.

8. An organic electroluminescent device according to any one of claims 1 to 6, wherein the light-emitting dopant material comprises a thermally activated delayed fluorescent light-emitting dopant material.

9. An organic electroluminescent device according to claim 1, wherein a difference in electron affinity (EA) between the first host and the second host is more than 0.1 eV and less than 0.6 eV.

10. An organic electroluminescent device according to claim 1, further comprising a hole-blocking layer adjacent to the light-emitting layers, the hole-blocking layer containing a compound represented by the general formula (1).

## Patentansprüche

1. Eine organische Elektrolumineszenzvorrichtung, umfassend eine oder mehrere lichtemittierende Schichten zwischen einer Anode und einer Kathode, welche einander gegenüberliegen, wobei mindestens eine der lichtemittierenden Schichten einen ersten Host, ausgewählt aus Verbindungen, jeweils dargestellt durch die folgende allgemeine Formel (1), einen zweiten Host, ausgewählt aus Verbindungen, jeweils dargestellt durch die folgende allgemeine Formel (2), und ein lichtemittierendes Dotierungsmaterial enthält: 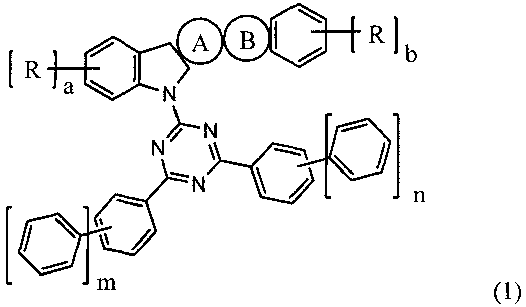 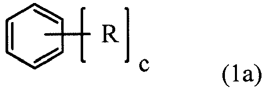 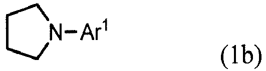 wobei
ein Ring A einen durch die Formel (1a) dargestellten aromatischen Kohlenwasserstoffring umfasst, ein Ring B einen durch die Formel (1b) dargestellten Heterocyclus umfasst, und der Ring A und der Ring B jeweils mit einem benachbarten Ring an beliebigen Positionen kondensiert sind;
Ar¹ eine Phenylgruppe, eine Biphenylgruppe oder eine Terphenylgruppe darstellt;
die Reste R jeweils unabhängig eine aliphatische Kohlenwasserstoffgruppe mit 1 bis 10 Kohlenstoffatomen, eine aromatische Kohlenwasserstoffgruppe mit 6 bis 10 Kohlenstoffatomen oder eine aromatische heterocyclische Gruppe mit 3 bis 12 Kohlenstoffatomen darstellen;
a, b und c jeweils unabhängig eine ganze Zahl von 0 bis 3 darstellen; und
m und n jeweils unabhängig eine ganze Zahl von 0 bis 2 darstellen und m+n eine ganze Zahl von 1 oder mehr darstellt; 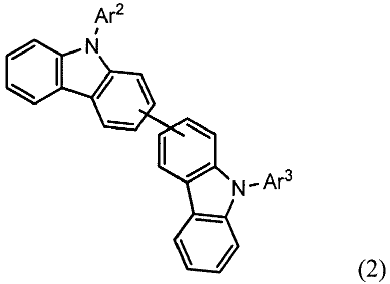
wobei Ar² und Ar³ jeweils unabhängig eine aromatische Kohlenwasserstoffgruppe mit 6 bis 14 Kohlenstoffatomen oder eine Gruppe, erhalten durch Verbinden von zwei oder drei der aromatischen Kohlenwasserstoffgruppen, darstellt und mindestens eines von Ar² oder Ar³ eine kondensierte aromatische Kohlenwasserstoffgruppe darstellt.

2. Eine organische Elektrolumineszenzvorrichtung gemäß Anspruch 1, wobei die Verbindung dargestellt durch die allgemeine Formel (2) eine durch die folgende allgemeine Formel (3) dargestellte Verbindung umfasst: 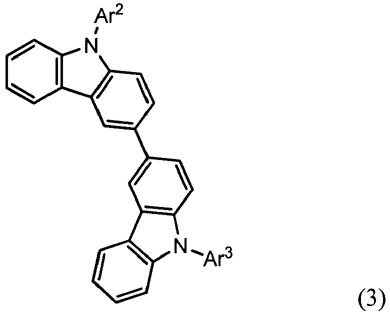 wobei Ar² und Ar³ die gleiche Bedeutung wie Ar² und Ar³ der allgemeinen Formel (2) haben.

3. Eine organische Elektrolumineszenzvorrichtung gemäß Anspruch 1, wobei Ar² eine Naphthylgruppe oder eine Phenanthrylgruppe darstellt.

4. Eine organische Elektrolumineszenzvorrichtung gemäß Anspruch 1, wobei die organische Elektrolumineszenzvorrichtung eine lichtemittierende Schicht umfasst, erhalten durch Aufbringen eines Hostmaterials, enthaltend ein vorläufiges Gemisch des ersten Hosts und des zweiten Hosts, durch Gasphasenabscheidung.

5. Eine organische Elektrolumineszenzvorrichtung gemäß Anspruch 1, wobei ein Unterschied in der Temperatur, bei der die Gewichtsreduktion 50% beträgt, zwischen dem ersten Host und dem zweiten Host 20°C oder weniger beträgt.

6. Eine organische Elektrolumineszenzvorrichtung gemäß Anspruch 1, wobei ein Verhältnis des ersten Hosts mehr als 20 Gew.-% und weniger als 55 Gew.-%, bezogen auf eine Gesamtmenge des ersten Hosts und des zweiten Hosts, beträgt.

7. Eine organische Elektrolumineszenzvorrichtung gemäß einem der Ansprüche 1 bis 6, wobei das lichtemittierende Dotierungsmaterial einen metallorganischen Komplex, enthaltend mindestens ein Metall, ausgewählt aus der Gruppe bestehend aus Ruthenium, Rhodium, Palladium, Silber, Rhenium, Osmium, Iridium, Platin und Gold, umfasst.

8. Eine organische Elektrolumineszenzvorrichtung gemäß einem der Ansprüche 1 bis 6, wobei das lichtemittierende Dotierungsmaterial ein thermisch aktiviertes, verzögert fluoreszierendes, lichtemittierendes Dotierungsmaterial umfasst.

9. Eine organische Elektrolumineszenzvorrichtung gemäß Anspruch 1, wobei eine Differenz in der Elektronenaffinität (EA) zwischen dem ersten Host und dem zweiten Host mehr als 0,1 eV und weniger als 0,6 eV beträgt.

10. Eine organische Elektrolumineszenzvorrichtung gemäß Anspruch 1, ferner umfassend eine lochblockierende Schicht angrenzend an die lichtemittierenden Schichten, wobei die lochblockierende Schicht eine Verbindung, dargestellt durch die allgemeine Formel (1), enthält.

## Revendications

1. Dispositif électroluminescent organique, comprenant une ou plusieurs couches luminescentes entre une anode et une cathode opposées l'une à l'autre, dans lequel au moins l'une des couches luminescentes contient un premier hôte choisi parmi les composés représentés chacun par la formule générale (1) suivante, un deuxième hôte choisi parmi les composés représentés chacun par la formule générale (2) suivante, et un matériau dopant luminescent : 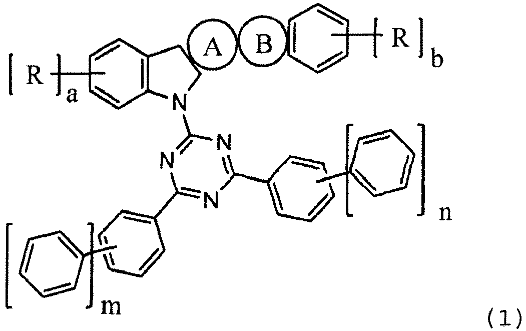 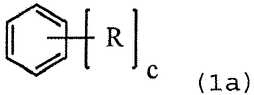 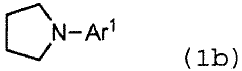 où
le cycle A comprend un cycle hydrocarboné aromatique représenté par la formule (la), le cycle B comprend un hétérocycle représenté par la formule (1b), et le cycle A et le cycle B sont condensés chacun avec un cycle adjacent en des positions arbitraires ;
Ar¹ représente un groupe phényle, un groupe biphényle, ou un groupe terphényle ;
chaque R représente indépendamment un groupe hydrocarboné aliphatique ayant 1 à 10 atomes de carbone, un groupe hydrocarboné aromatique ayant 6 à 10 atomes de carbone, ou un groupe hétérocyclique aromatique ayant 3 à 12 atomes de carbone ;
chacun de a, b et c représente indépendamment un entier de 0 à 3 ; et
chacun de m et n représente indépendamment un entier de 0 à 2, et m+n représente un entier de 1 ou plus ; 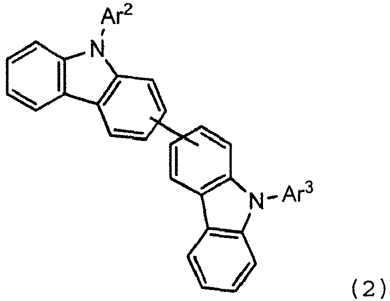
où chacun de Ar² et Ar³ représente un groupe hydrocarboné aromatique ayant 6 à 14 atomes de carbone, ou un groupe obtenu par liaison de deux ou trois des groupes hydrocarbonés aromatiques, et au moins l'un parmi Ar² et Ar³ représente un groupe hydrocarboné aromatique condensé.

2. Dispositif électroluminescent organique selon la revendication 1, dans lequel le composé représenté par la formule générale (2) comprend un composé représenté par la formule générale (3) suivante : 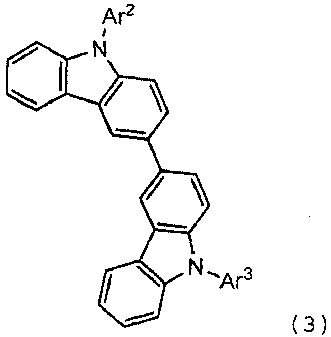 dans laquelle Ar² et Ar³ ont des significations identiques à celles de Ar² et Ar³ dans la formule générale (2).

3. Dispositif électroluminescent organique selon la revendication 1, dans lequel Ar² représente un groupe naphtyle ou un groupe phénanthryle.

4. Dispositif électroluminescent organique selon la revendication 1, dans lequel le dispositif électroluminescent organique comprend une couche luminescente obtenue par déposition en phase vapeur d'un matériau hôte contenant un mélange préliminaire du premier hôte et du deuxième hôte.

5. Dispositif électroluminescent organique selon la revendication 1, dans lequel la différence de température pour une réduction de poids de 50 % entre le premier hôte et le deuxième hôte est de 20 °C ou moins.

6. Dispositif électroluminescent organique selon la revendication 1, dans lequel la proportion du premier hôte est supérieure à 20 % en poids et inférieure à 55 % en poids par rapport au total du premier hôte et du deuxième hôte.

7. Dispositif électroluminescent organique selon l'une quelconque des revendications 1 à 6, dans lequel le matériau dopant luminescent comprend un complexe organométallique contenant au moins un métal choisi dans l'ensemble constitué par le ruthénium, le rhodium, le palladium, l'argent, le rhénium, l'osmium, l'iridium, le platine, et l'or.

8. Dispositif électroluminescent organique selon l'une quelconque des revendications 1 à 6, dans lequel le matériau dopant luminescent comprend un matériau dopant luminescent fluorescent différé activé thermiquement.

9. Dispositif électroluminescent organique selon la revendication 1, dans lequel la différence d'affinité électronique (EA) entre le premier hôte et le deuxième hôte est supérieure à 0,1 eV et inférieure à 0,6 eV.

10. Dispositif électroluminescent organique selon la revendication 1, comprenant en outre une couche de blocage de trous adjacente aux couches luminescentes, la couche de blocage de trous contenant un composé représenté par la formule générale (1).
